# EUROPEAN PATENT APPLICATION

(11) **EP 4 527 384 A2**
(43) Date of publication of application: **26.03.2025**
(21) Application number: 24215754.3
(22) Date of filing: 16.04.2021
(51) Int. Cl.: A61K 31/4015

(54) **WATER-SOLUBLE, MODIFIED AMINO ACID DERIVATIVES FOR THE TREATMENT OF NEUROLOGICAL DISEASES AND SELECTED PSYCHIATRIC DISORDERS**

(30) Priority: 16.04.2020 PL 43356720
(62) Divisional of application: 21732998.6
(71) Applicant: UNIWERSYTET JAGIELLONSKI, 31-007 Kraków (PL); Warszawski Uniwersytet Medyczny, 02-091 Warszawa (PL)
(72) Inventor: KAMINSKI, Krzysztof, 30-694 Kraków (PL); ABRAM, Michal, 30-816 Kraków (PL); JAKUBIEC, Marcin, 43-365 Wilkowice (PL); RAPACZ, Anna, 30-611 Kraków (PL); MOGILSKI, Szczepan, 33-336 Labowa (PL); LATACZ, Gniewomir, 32-340 Wolbrom (PL); STRUGA, Marta, 05-822 Milanówek (PL)
(74) Representative: Haseltine Lake Kempner LLP

(57) **Abstract**

Water-soluble analogs of compounds intended for the treatment of neurological diseases, epilepsy, neuropathic pain and migraine, and psychiatric disorders, including anxiety and depression, especially suitable for the preparation of oral or parenteral drug forms, are disclosed.

## Description

### Field of the invention

The invention relates to chemical compounds, which concerning their structure, are water-soluble, modified amino acids derivatives.

The compounds disclosed in the present application are close analogs of substances known in the art, and are intended for the treatment of neurological disorders(epilepsy, neuropathic pain, and migraine), as well as psychiatric disorders, e.g. anxiety and depression. In comparison to their precursors, the compounds according to the present invention are characterized by more favorable physical and biopharmaceutical properties, i.e. by better water solubility, therefore, they can be used as active substances in medicinal preparations. The good water solubility of the disclosed compounds is beneficial for the development of both oral and parenteral dosage forms.

### Background of the invention

The foregoing studies on pyrrolidine-2,5-dione derivatives as candidates for new antiepileptic drugs, has revealed that compounds with alanine as the central part of the molecule, have particularly favorable pharmacological properties and a high safety margin. The beneficial chemical modifications of the said amino acid relied on incorporation of alanine amino group into a succinimide ring, as well as substitution of the carboxylic group of the titled amino acid, with a benzylamine moiety (Kamiński, et al. Bioorg. Med. Chem. 2015, 23, 2548-2561; Rapacz, et al. Naunyn Schmiedeberg's Arch. Pharmacol. 2017, 6, 567-579). Among these derivatives, compounds with an aromatic ring which is unsubstituted or substituted with a fluorine atom in the 2-position have particularly favorable biological properties, wherein, compounds with the absolute *R*-configuration demonstrate the most potent activity (substances disclosed in Polish patent application no. P.429656). Importantly, this molecules in anticonvulsant effective doses increase the spontaneous locomotor activity of mice, which excludes their sedative effect, which is one of the most common adverse effects of antiepileptic drugs currently in pharmacotherapy. The stimulating effect was not observed for the *S*-enantiomer and the racemic mixture (*R,S*). The known compounds show a broad protective activity in animal models of epileptic convulsion, and in the case of *R*-enantiomers, their effectiveness has also been proven in animal models of neuropathic pain, and in the model of depression and anxiety. The general structures of disclosed compounds are illustrated below.

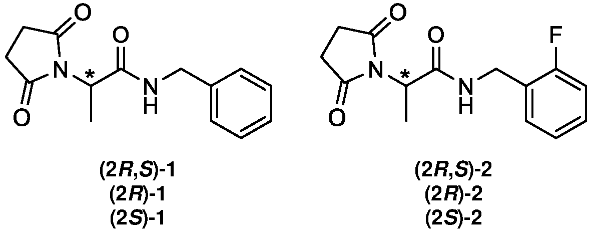

### Structures of compounds known in state of the art

Despite the favorable biological data for the aforementioned substances, in particular for the compounds with the absolute *R*-configuration - **(*2R*)-1** and **(*2R*)-2,** their unsatisfactory water solubility may cause a serious problem in further clinical development of the compounds. It should be stressed, that water solubility of compound is one of the most important physical properties of drug-candidates, especially in terms of absorption from the gastrointestinal tract, ease of preparation of the appropriate formulation of the drug, and the possibility of parenteral (primarily intravenous) administration of the preparation. Poor water solubility of active ingredient is also the main reason of low pharmaceutical and/or biological availability after oral administration, which often leades to discontinuation of the development of a new drug candidate in the preclinical and clinical trials. Moreover, literature data indicate that nearly 70% of drug candidates are poorly soluble in water (Khadka et al. J. Pharm. Sci. 2014, 9, 304-316). Importantly, in the case of substance with low water solubility, the therapeutic concentration in the blood after oral administration is usually achieved by increasing the dose used. However, such solution causes a risk of local gastrointestinal toxicity, systemic toxicity, and increases the likelihood of drug interactions. Therefore, good water solubility has been identified as one of the key physical properties taken into account in the search and development of new drug candidates. (Vo et al. Eur. J. Pharm. Biopharm. 2013, 85, 799-813; Kawabata et al. Int. J. Pharm. 2011, 420, 1-10). The basic methods used to improve the solubility of substances in water include physical methods (e.g. reduction of particle size by micronization, modification of the crystal structure, formation of eutectic mixtures or solid dispersions of the drug in the carrier, etc.), and chemical methods, such as structural modifications aimed at increase of polarity of the compound, salt, or prodrug formation (Göke et al. Eur. J. Pharm. Biopharm. 2018, 126, 40-56; Jermain et al. Int. J. Pharm. 2018, 535, 379-392). Considering the above facts, the technical problem solved by the present invention is to provide substances that are close analogues of the aforementioned known in the state of art lead compounds, in particular with the R-configuration of the stereogenic center in the amino acid moiety, but with much more favorable physical and biopharmaceutical properties, i.e. good solubility in water. The compounds according to the invention, due to slight structural modifications and satisfactory water solubility, should show similar biological properties, as the parent compounds. In addition, the improved water solubility will also be beneficial for the development of oral dosage forms, and will enable parenteral (including intravenous) administration of the disclosed compounds.

### Summary of the invention

The invention relates to a compound of general formula **I:** 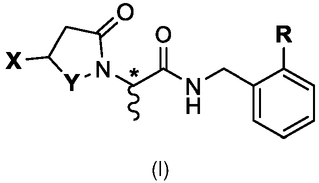 wherein:
**X** represents hydrogen or N(CH₃)₂,
**Y** represents CH₂ or C=O,
**R** represents hydrogen or a halogen atom, preferably F,
wherein, when **Y** is C=O, then **X** represents N(CH₃)₂,
or its optical isomers, mixtures thereof, and pharmaceutically acceptable salts.

Preferably, the compound according to the invention is selected from the group comprising: *N*-benzyl-2-(2-oxopyrrolidin-1-yl)propanamide
*N*-(2-fluorobenzyl)-2-(2-oxopyrrolidin-1-yl)propanamide,
*N*-benzyl-2-(3-(dimethylamino)-2,5-dioxopyrrolidin-1-yl)propanamide
and 2-(3-(dimethylamino)-2,5- dioxopyrrolidin-1-yl)-*N*-(2-fluorobenzyl)propanamide.

More preferably, the compound according to the invention is selected from the group comprising: *N-*benzyl-2-(3-(dimethylamino)-2,5-dioxopyrrolidin-1-yl)propanamide, and 2-(3-(dimethylamino)-2,5-ioxopyrrolidin-1-yl)-*N*-(2-fluorobenzyl)propanamide.

Preferably, the compound according to the invention, is in a form of pharmaceutically acceptable salt, selected from: hydrochloride, sulfate, methanesulfonate, toluenesulfonate, succinate, fumarate, or lactate.

Preferably, the compound according to the invention is selected from the group comprising:
(*2R*)-*N*-benzyl-2-(2-oxopyrrolidin-1-yl)propanamide,
(*2S*)-*N*-benzyl-2-(2-oxopyrrolidin-1-yl)propanamide,
(*2R*,*S*)-*N*-benzyl-2-(2-oxopyrrolidin-1-yl)propanamide,
(*2R*)-*N*-(2-fluorobenzyl)-2-(2-oxopyrrolidin-1-yl)propanamide,
(*2S*)-*N*-(2-fluorobenzyl)-2-(2-oxopyrrolidin-1-yl)propanamide,
(*2R,S*)-*N*-(2-fluorobenzyl)-2-(2-oxopyrrolidin-1-yl)propanamide,
(*2R*)-*N*-benzyl-2-(3-(dimethylamino)-2,5-dioxopyrrolidin-1-yl)propanamide hydrochloride,
(*2S*)-*N*-benzyl-2-(3-(dimethylamino)-2,5-dioxopyrrolidin-1-yl)propanamide hydrochloride,
(*2R*,*S*)-*N*-benzyl-2-(3-(dimethylamino)-2,5-dioxopyrrolidin-1-yl)propanamide hydrochloride,
(*2R*)-2-(3-(dimethylamino)-2,5-dioxopyrrolidin-1-yl)-*N*-(2-fluorobenzyl)propanamide hydrochloride,
(*2S*)-2-(3-(dimethylamino)-2,5-dioxopyrrolidin-1-yl)-*N*-(2-fluorobenzyl)propanamide hydrochloride,
(*2R,S*)-2-(3-(dimethylamino)-2,5-dioxopyrrolidin-1-yl)-*N*-(2-fluorobenzyl)propanamide hydrochloride,
(*2R*)-*N*-benzyl-2-(3-(dimethylamino)-2,5-dioxopyrrolidin-1-yl)propanamide metanesulfonate,
(*2S*)-*N*-benzyl-2-(3-(dimethylamino)-2,5-dioxopyrrolidin-1-yl)propanamide metanesulfonate,
(*2R,S*)-*N*-benzyl-2-(3-(dimethylamino)-2,5-dioxopyrrolidin-1-yl)propanamide metanesulfonate,
(*2R*)-*N*-benzyl-2-(3-(dimethylamino)-2,5-dioxopyrrolidin-1-yl)propanamide toluenesulfonate,
(*2S*)-*N*-benzyl-2-(3-(dimethylamino)-2,5-dioxopyrrolidin-1-yl)propanamide toluenesulfonate,
(*2R,S*)-*N*-benzyl-2-(3-(dimethylamino)-2,5-dioxopyrrolidin-1-yl)propanamide toluenesulfonate,
(*2R*)-*N*-benzyl-2-(3-(dimethylamino)-2,5-dioxopyrrolidin-1-yl)propanamide lactate,
(*2S*)-*N*-benzyl-2-(3-(dimethylamino)-2,5-dioxopyrrolidin-1-yl)propanamide lactate,
(*2R,S*)-*N*-benzyl-2-(3-(dimethylamino)-2,5-dioxopyrrolidin-1-yl)propanamide lactate,
(*2R*)-*N*-benzyl-2-(3-(dimethylamino)-2,5-dioxopyrrolidin-1-yl)propanamide sulfate,
(*2S*)-*N*-benzyl-2-(3-(dimethylamino)-2,5-dioxopyrrolidin-1-yl)propanamide sulfate,
(*2R,S*)-*N*-benzyl-2-(3-(dimethylamino)-2,5-dioxopyrrolidin-1-yl)propanamide sulfate,
(*2R*)-*N*-benzyl-2-(3-(dimethylamino)-2,5-dioxopyrrolidin-1-yl)propanamide succinate,
(*2S*)-*N*-benzyl-2-(3-(dimethylamino)-2,5-dioxopyrrolidin-1-yl)propanamide succinate,
(*2R,S*)-*N*-benzyl-2-(3-(dimethylamino)-2,5-dioxopyrrolidin-1-yl)propanamide succinate,
(*2R*)-*N*-benzyl-2-(3-(dimethylamino)-2,5-dioxopyrrolidin-1-yl)propanamide fumarate,
(*2S*)-*N*-benzyl-2-(3-(dimethylamino)-2,5-dioxopyrrolidin-1-yl)propanamide fumarate,
(*2R,S*)-*N*-benzyl-2-(3-(dimethylamino)-2,5-dioxopyrrolidin-1-yl)propanamide fumarate,
(*2R*)-2-(3-(dimethylamino)-2,5-dioxopyrrolidin-1-yl)-*N*-(2-fluorobenzyl)propanamide methanesulfonate,
(*2S*)-2-(3-(dimethylamino)-2,5-dioxopyrrolidin-1-yl)-*N*-(2-fluorobenzyl)propanamide methanesulfonate,
(*2R,S*)-2-(3-(dimethylamino)-2,5-dioxopyrrolidin-1-yl)-*N*-(2-fluorobenzyl)propanamide methanesulfonate,
(*2R*)-2-(3-(dimethylamino)-2,5-dioxopyrrolidin-1-yl)-*N*-(2-fluorobenzyl)propanamide toluenesulfonate,
(*2S*)-2-(3-(dimethylamino)-2,5-dioxopyrrolidin-1-yl)-*N*-(2-fluorobenzyl)propanamide toluenesulfonate,
(*2R,S*)-2-(3-(dimethylamino)-2,5-dioxopyrrolidin-1-yl)-*N*-(2-fluorobenzyl)propanamide toluenesulfonate,
(*2R*)-2-(3-(dimethylamino)-2,5-dioxopyrrolidin-1-yl)-*N*-(2-fluorobenzyl)propanamide lactate,
(*2S*)-2-(3-(dimethylamino)-2,5-dioxopyrrolidin-1-yl)-*N*-(2-fluorobenzyl)propanamide lactate,
(*2R,S*)-2-(3-(dimethylamino)-2,5-dioxopyrrolidin-1-yl)-*N*-(2-fluorobenzyl)propanamide lactate,
(*2R*)-2-(3-(dimethylamino)-2,5-dioxopyrrolidin-1-yl)-*N*-(2-fluorobenzyl)propanamide sulfate,
(*2S*)-2-(3-(dimethylamino)-2,5-dioxopyrrolidin-1-yl)-*N*-(2-fluorobenzyl)propanamide sulfate,
(*2R,S*)-2-(3-(dimethylamino)-2,5-dioxopyrrolidin-1-yl)-*N*-(2-fluorobenzyl)propanamide sulfate,
(*2R*)-2-(3-(dimethylamino)-2,5-dioxopyrrolidin-1-yl)-*N*-(2-fluorobenzyl)propanamide succinate,
(*2S*)-2-(3-(dimethylamino)-2,5-dioxopyrrolidin-1-yl)-*N*-(2-fluorobenzyl)propanamide succinate,
(*2R,S*)-2-(3-(dimethylamino)-2,5-dioxopyrrolidin-1-yl)-*N*-(2-fluorobenzyl)propanamide succinate,
(*2R*)-2-(3-(dimethylamino)-2,5-dioxopyrrolidin-1-yl)-*N*-(2-fluorobenzyl)propanamide fumarate,
(*2S*)-2-(3-(dimethylamino)-2,5-dioxopyrrolidin-1-yl)-*N*-(2-fluorobenzyl)propanamide fumarate, and
(*2R,S*)-2-(3-(dimethylamino)-2,5-dioxopyrrolidin-1-yl)-*N*-(2-fluorobenzyl)propanamide fumarate.

In a preferred embodiment, the invention relates to a water soluble derivative of pyrrolidin-2-one, or pyrrolidin-2,5-dione, selected from the following compounds: *N*-benzyl-2-(2-oxopyrrolidin-1-yl)propanamide (**3**), *N*-(2-fluorobenzyl)-2-(2-oxopyrrolidin-1-yl)propanamide (**4**), *N*-benzyl-2-(3-(dimethylamino)-2,5-dioxopyrrolidin-1-yl)propanamide (**5**), and 2-(3-dimethylamino)-2,5-dioxopyrrolidin-1-yl)-*N*-(2-fluorobenzyl)propanamide (**6**), whose general structure is shown below: 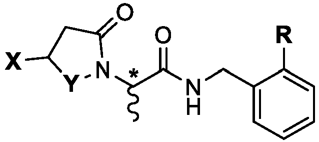 **wherein:**
compound **3: X**=H, **Y**= CH₂, **R**=H
compound **4: X**=H, **Y**= CH₂, **R**=F
compound **5: X**=N(CH₃)₂, **Y**= C=O, **R**=H
compound **6: X**=N(CH₃)₂, **Y**= C=O, **R**=F

Due to the presence of the tertiary amino group in the 3-position of the imide ring, in the structure of the compounds **5** and **6,** these derivatives form water-soluble salts. Pharmaceutically acceptable salts include, but are not limited to, hydrochlorides, sulfates, methanesulfonates, toluenesulfonates, succinates, fumarates, lactates, etc. These salts, as well as other pharmaceutically acceptable salt-like compositions also constitute the subject of the present invention. According to the solubility classification proposed by the European Pharmacopoeia 10.0 and the Polish Pharmacopoeia XII, the compounds that are the subject of the present invention are classified as water-soluble substances (compounds **3** and **4**) or as substances easily soluble in water (compounds **5** and **6**). The compound of formula **I** has a chiral center in the propanamide moiety, hence it can exist in the form of optical isomers and mixtures thereof. The said optical isomers, and their mixtures at various ratios, including the racemic mixtures, are within the scope of the invention.

### Detailed description of the invention

The invention relates to water-soluble derivatives of 2-(2-oxopyrrolidin-1-yl)propanamide or (3-dimethylamino)-2,5-dioxopyrrolidin-1-yl)propanamide.

The compounds were designed as close structural analogs of the compounds disclosed previously and known in the state of art (**Fig. 1**), and the main chemical modifications relies on the removal of one carbonyl group, leading to pyrrolidin-2-one derivatives (compounds **3** and **4**) or on introduction of the dimethylamine moiety at the 3-position of the pyrrolidine-2,5-dione ring (compounds **5** and **6**). Due to the presence of the said amino group, it is possible to transform the compounds into water-soluble and pharmaceutically acceptable salts, including hydrochlorides, sulfates, methanesulfonates, toluenesulfonates, succinates, fumarates, lactates, etc., by application of methods known in the art. According to the invention, compounds of formula **3-6** can be prepared in a multi-step process, starting from commercially available, *Tert*-butoxycarbonyl (Boc) protected D,L-alanine (in the case of the racemic (*R,S*) mixtures), D-alanine (in the case of the compounds with the absolute *R*-configuration in the propanamide moiety), or L-alanine (in the case of the enantiomers with the absolute S-configuration in the propanamide moiety). Alternatively, the *R*- and S-enantiomers can by prepared by separation of suitable racemic mixtures, using the separation methods known in the art.

The first step of the synthesis consists of condensation of benzylamine or 2-fluorobenzylamine with Boc protected alanine, having the desired configuration of the asymmetric center, to give the intermediate of formula **II.** Next, removing of the Boc protected group leads to the starting primary amine of general formula **III.** The steps *i* i *ii* are common for the compound **3-6.** In case of synthesis of pyrrolidin-2-one derivatives **3** and **4,** the primary amine **III** is acylated using 4-chlorobutanoic acid chloride to yield the derivative **IV.** Next, compound **IV** is cyclized in the presence of base, e.g. sodium hydride, to give the desired products **3** and **4.** Alternatively, instead of 4-chlorobutanoic acid chloride, 4-bromo or 4-iodobutanoic acid chlorides could be used. The synthesis of compounds **3** and **4** is shown in **Scheme 1.**

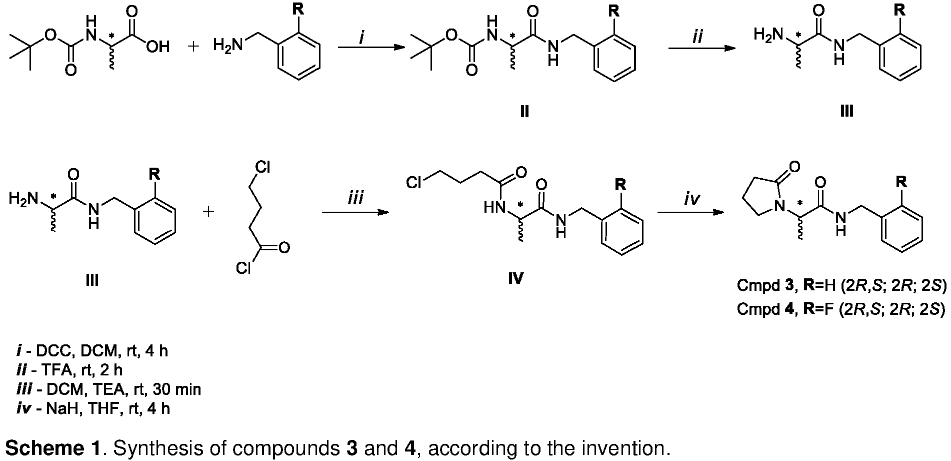

In the case of salt derivatives **5, 6,** the amine **III** is condensed with maleic anhydride to give monounsaturated amido acid **V**, which is next cyclized to maleimide derivative **VI.** Addition reaction of dimethylamine to the double bond of the compound **VI,** results in formation of tertiary amine, which next can be transformed into its water soluble salts, using the methods known in the art. Pharmaceutically acceptable salts include, among others, hydrochlorides, sulfates, methanesulfonates, toluenesulfonates, succinates, fumarates, lactates, etc. As examples, compounds **5** and **6** were obtained in the form of all the aforementioned salts. The subsequent steps of the synthesis of compounds **5** and **6** are demonstrated in **Scheme 2.**

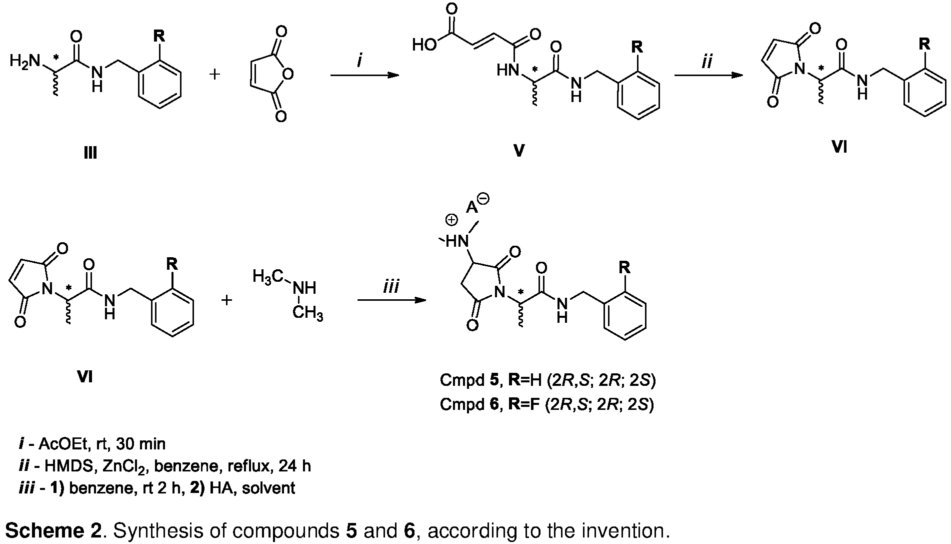

An advantageous feature of the compounds **3-6,** in comparison to their chemical prototypes **1** and **2** is good water solubility, which may result in better pharmacokinetics, particularly in better pharmaceutical availability, better absorption from the gastrointestinal tract, and higher bioavailability. In addition, good solubility in water facilitates preparation of an appropriate oral form of the drug, and enable parenteral administration of the preparation, which is especially desirable in emergency situations, when a quick therapeutic effect is needed, e.g. to stop an epileptic seizure. Therefore, derivatives **3-6** should be regarded as close analogs of the lead compounds **1** and **2,** but having more favorable physical and biopharmaceutical properties, i.e. better water solubility. **Table 1** summarizes the results of the water solubility studies, per gram of compound at 25°C. The studies were performed for racemic mixtures (*R,S*), as well as for individual *R-* or *S*-enantiomers.

**Table 1. Results of water solubility test, at 25°C, per 1 gram of compound.**

| Compound | The volume of water required for dissolution of 1 g of the substance at 25 ºC (mL) ^{a} | Matching term^{b} |
|---|---|---|
| **(2*R*,*S*)-1** | > 100^{c} | Slightly soluble |
| **(2*R*)-1** | > 100^{c} | Slightly soluble |
| **(2*S*)-1** | > 100^{c} | Slightly soluble |
| **(2*R*,*S*)-2** | > 100^{c} | Slightly soluble |
| **(2*R*)-2** | > 100^{c} | Slightly soluble |
| **(2*S*)-2** | > 100^{c} | Slightly soluble |
| **(2*R*,*S*)-3** | 25 | Soluble |
| **(2*R*)-3** | 25 | Soluble |
| **(2*S*)-3** | 25 | Soluble |
| **(2*R*,*S*)-4** | 30 | Soluble |
| **(2*R*)-4** | 30 | Soluble |
| **(2*S*)-4** | 30 | Soluble |
| **(2*R*,*S*)-5 × HCl** | <1 | Very soluble |
| **(2*R*)-5 × HCl** | <1 | Very soluble |
| **(2*S*)-5 × HCl** | <1 | Very soluble |
| **(2*R*,*S*)-6 × HCl** | <1 | Very soluble |
| **(2*R*)-6 × HCl** | <1 | Very soluble |
| **(2*S*)-6 × HCl** | <1 | Very soluble |

| | | |
|---|---|---|
| ^{a} The studies were performed in accordance with the guidelines described in the European Pharmacopoeia 10.0 and the Polish Pharmacopoeia XII. ^{b} Terminology in accordance with the European Pharmacopoeia 10.0 and the Polish Pharmacopoeia XII. ^{c} Maximum volume of the used water - 100 mL. | | |

The obtained results indicate that the compounds disclosed in this application are classified as substances that are soluble (**3, 4**) or easily soluble (**5, 6**) in water, while the starting compounds (**1** and **2**), according to the accepted classification, rank as substances that are at least hardly soluble. It should be emphasized, that the better solubility in water is particularly surprising and not obvious in the case of pyrrolidin-2-one derivatives (**3, 4**) vs. pyrrolidine-2,5-dione derivatives (**1, 2**). The physicochemical data calculated using the SwissADME program (Daina et al. Sci. Rep. 2017, 7, 42717) indicate lower lipophilicity and, at the same time, greater polar surface area of the molecule for succinimide derivatives (**1, 2**), which suggests their potentially better solubility in water. Experimental data revealed the opposite effect, i.e. a much better water solubility for pyrrolidin-2-one derivatives (**3, 4**). **Table 2** summarizes the values of lipophilicity parameters (log *P*_{o/w}) and total polar surface area (tPSA) for racemic mixtures of pyrrolidine-2,5-dione derivatives - **(2*R*,*S*)-1, (2*R*,*S*)-2** vs. pyrrolidin-2-one derivatives - **(2*R*,*S*)-3** and **(2*R,S*)-4.**

**Table 2. Comparison of lipophilicity parameters (log P_{o/w}) and total polar surface area (tPSA) for racemic mixtures of pyrrolidine-2,5-dione derivatives (2R,S)-1, (2R,S)-2) and pyrrolidin-2-one derivatives (2R,S)-3 and (2R,S)-4).**

| Compound | Physicochemical parameter^{a} | |
|---|---|---|
| | Log *P*_{o/w}^{b} | tPSA [Å²]^{c} |
| **(2*R,S*)-1** | 1.11 | 66.48 |
| **(2*R,S*)-2** | 1.42 | 66.48 |
| **(2*R,S*)-3** | 1.44 | 49.41 |
| **(2*R,S*)-4** | 1.76 | 49.41 |

| | | |
|---|---|---|
| ^{a} The calculations were made with the use of the computer program SwissADME (Daina et al. Sci. Rep. 2017, 7, 42717). ^{b} Log *P*_{o/w} - logarithm of the n-octanol/water partition coefficient. ^{c} tPSA - total polar surface area of the molecule. | | |

Examples of realization of the invention are presented below.

Example 1. Preparation of compounds according to the invention.

### Analytical methods:

The ¹H NMR and ¹³C NMR spectra were recorded using JEOL-500 spectrometer (JEOL USA, Inc. MA, USA), at 500 MHz and 126 MHz, respectively. Chemical shifts are given in δ (ppm) values relative to TMS δ = 0 (¹H) as internal standard. *J* values are expressed in Hertz (Hz). Deuterated chloroform (CDCl₃) or deuterated dimethylsulfoxide (DMSO-d6) were used as solvents. The following signal abbreviations were used in the spectra description: s (singlet), br. s (broad singlet), d (doublet), t (triplet), q (quartet), m (multiplet). The UPLC/MS analysis system consisted of a Waters ACQUITY^{®} UPLC^{®} instrument (Waters Corporation, Milford, MA, USA) coupled to a Waters TQD mass spectrometer operating in electrospray ionization (ESI) mode. Chromatographic separations were carried out using the Acquity UPLC BEH C18 column of dimensions 2.1 × 100 mm and grain diameter of 1.7 µm. The column was kept at 40°C, and eluted with a gradient from 95% to 0% of eluent A over 10 min, with a flow of 0.3 ml/min. Eluent A: water/formic acid (0.1 %, *v*/*v*); eluent B: acetonitrile/formic acid (0.1 %, *v*/*v).* The chromatograms were recorded using a Waters eλ PDA detector. Spectra were analyzed in the range of 200-700 nm with a resolution of 1.2 nm, and a sampling rate of 20 points/s. The enantiomeric purity was determined by chiral HPLC analysis using a Shimadzu Prominence and LC-2030C SD Plus apparatus, (Shimadzu Corporation, Kyoto, Japan) equipped with an Amylose-C chiral column (250 × 4.6 mm). The analysis was performed under the following conditions: column temperature: 20°C, mixture of eluents: hexane/i-PrOH = 85/15 (*v*/*v*), flow: 0.7 mL/min, detection at λ = 209 nm. For intermediates **VI,** the analyses were performed under the following conditions: column temperature - 33°C, mixture of hexane/i-PrOH/TFA eluents = 93.4/6.4/0.2 (*v*/*v*/*v*), flow: 0.75 mL/min, detection at λ = 206 nm. Thin layer chromatography (TLC) was performed on aluminum plates coated with silica gel 60 F₂₅₄ (Macherey-Nagel, Düren, Germany) using developing systems having the following composition: DCM : MeOH (9 : 0.3; *v*/*v*), DCM : MeOH (9 : 0.5; *v*/*v).* Spot detection - UV light (λ = 254 nm). The melting points (mp) were determined using open capillaries and Büchi 353 apparatus (Büchi Labortechnik, Flawil, Switzerland). Names of the compounds described below and illustrating embodiments of the invention, were generated using the ChemBioDraw Ultra 12.0 program.

The preparation of the compounds according to the invention is described in the Examples below. The presented syntheses were not optimized in terms of yield, amount of the reagents used, and the final form of the obtained compounds.

Abbreviations used: AcOEt - ethyl acetate, DCM - dichloromethane, DCC - *N,N'-*dicyclohexylcarbodiimide, Et₂O - diethyl ether, HCl - hydrochloric acid, HMDS - hexamethyldisilazane, MeOH - methanol, NaCl - sodium chloride, NaH - sodium hydride, NH₄OH - ammonium hydroxide, Na₂SO₄ - sodium sulfate, TFA - trifluoroacetic acid, TEA - triethylamine, ZnCl₂- zinc chloride.

### Examples of synthesis, physicochemical and spectral data of intermediates (II-IV), according to Scheme 1):

### Intermediate II (wherein R=H): Tert-butyl-(R)-(1-benzylamino)-1-oxopropan-2-yl)carbamate

Boc-D-alanine (5.1 g, 27 mmol, 1 eq) was dissolved in 20 mL of DCM, and DCC (6.68 g, 32.4 mmol, 1.2 eq) was added. The mixture was stirred for 30 min, next benzylamine (2.89 g, 27 mmol, 1 eq) was added dropwise, and the reaction was continued at room temperature for 4 h. Afterwards, DCM was distilled off, and the intermediate **II** was purified by column chromatography using DCM : MeOH (9 : 0.3; *v*/*v*) as eluent. The intermediate was obtained as a light oil. Yield 91% (6.95 g); TLC: R_{f} = 0.43 (DCM : MeOH (9 : 0.3; *v*/*v*)); C₁₅H₂₂N₂O₃ (278.35), monoisotopic mass: 278.16. UPLC (purity 100%): *t*_{R} = 5.44 min. (M+H)⁺ 279.3.

### Intermediate III (wherein R=H): (R)-2-amino-N-benzylpropanamide

TFA (10 mL) was added to a solution of *tert*-butyl-(*R*)-(1-(benzylamino)-1-oxopropan-2-yl)carbamate (6.95 g, 25 mmol, 1 eq) (**II**) in DCM (40 mL), and the mixture was stirred for 2 hours. The TFA was then neutralized with a 25% NH₄OH solution, and the mixture was extracted with DCM (3 × 50 mL). The organic layer was dried over anhydrous Na₂SO₄, and next DCM was evaporated to dryness. (R)-2-amino-*N*-benzylpropanamide (**III**) was obtained as a light oil. Yield: 89% (3.9 g); TLC: Rε = 0.21 (DCM : MeOH (9 : 0.5; *v*/*v*)); C₁₀H₁₄N₂O (178.24), monoisotopic mass: 178.11. UPLC (purity 96.8%): t_{R} = 2.11 min. (M + H)⁺ 179.2.

### Intermediate IV (wherein R= H) (R)-N-(1-(benzylamino)-1-oxopropan-2-yl)-4-chlorobutanamide

4-Chlorobutanoic acid chloride (0.59 g, 4.2 mmol, 1.5 eq), and TEA (0.85 g, 8.4 mmol, 3 eq) were added to a solution of (*R*)-2-amino-*N*-benzylpropanamide (**III**) (0.50 g, 2.8 mmol, 1 eq) in DCM (20 mL), and the mixture was stirred for 0.5 hour. Next, DCM was evaporated to dryness. The intermediate **IV** was purified by column chromatography using (DCM : MeOH (9 : 0.5; *v*/*v*) eluent system. The intermediate **IV** was obtained as a light oil. Yield: 82% (0.65 g); TLC: R_{f} = 0.53 (DCM : MeOH (9 : 0.5; *v*/*v*)); C₁₄H₁₉ClN₂O₂ (282.77), monoisotopic mass: 282.11. UPLC (purity 97.8%): t_{R} = 4.52 min. (M + H)⁺ 283.2.

### Synthesis , physicochemical and spectral data of the final compounds (2R)-3, (2S)-3, (2R,S)-3, and (2R)-4, (2S)-4, (2R,S)-4

### Compound (2R)-3 (wherein R=H): (2R)-N-benzyl-2-(2-oxopyrrolidin-1-yl)propanamide

NaH (0.106 g, 4.4 mmol, 2 eq) was added to a solution of (*R*)-*N*-(1-(benzylamino)-1-oxopropan-2-yl)-4-chlorobutanamide (0.63 g, 2.2 mmol, 1 eq) (**IV**, wherein **R**=H) in anhydrous THF. The reacton mixture was stirred for 4 hours, and next concentrated under reduced pressure. The oily residue was dissolved in 0.1 M HCl (50 mL) and extracted with DCM (3 × 50 mL). The organic layer was dried over anhydrous Na₂SO₄, and evaporated to dryness. The crude product was purified by column chromatography using DCM : MeOH (9 : 0.5; *vlv*) solvent system. After washing with Et₂O, the compound was obtained as a white solid. Yield: 86% (0.47 g); mp 96.7-97.5°C; Chiral HPLC > 99% ee (t_{R} = 10.623 min); TLC: R_{f} = 0.42 (DCM : MeOH (9 : 0.5; *v*/*v*)); C₁₄H₁₈N₂O₂ (246.31), Monoisotope mass: 264.13. UPLC (purity:> 99.9%): t_{R} = 3.92 min, (M+H)⁺ 247.2. ¹H NMR (500 MHz, CDCl₃) δ 1.36 (d, *J* = 7.2 Hz, 3H), 1.95-1.99 (m, 2H), 2.28-2.36 (m, 2H), 3.36-3.43 (m, 2H), 4.38 (dd, *J* = 5.9, 2.2 Hz, 2H), 4.65-4.74 (m, 1H), 6.75 (br. s, 1H), 7.19-7.22 (m, 2H), 7.23-7.25 (m, 1H), 7.27-7.31 (m, 2H). ¹³C NMR (126 MHz, CDCl₃) δ 13.8, 18.1, 31.1, 43.5, 43.8, 50.3, 127.5, 127.6, 128.7, 138.3, 170.6, 175.8.

### Compound (2S)-3 (wherein R=H): (2S)-N-benzyl-2-(2-oxopyrrolidin-1-yl)propanamide

The compound was obtained analogously to the compound **(2R)-3,** using (*S*)-*N*-(1-(benzylamino)-1-oxopropan-2-yl)-4-chlorobutanamide (0.63 g, 2.2 mmol, 1 eq) (**IV**, wherein **R**=H) and NaH (0.106 g, 4.4 mmol, 2 eq). The crude product was purified by column chromatography using DCM : MeOH (9 : 0.5; *v*/*v*) solvent system. After washing with Et₂O, the compound was obtained as a white solid. Yield: 79% (0.43 g); mp 96.4-97.2°C; Chiral HPLC > 99% ee (t_{R} = 14.215 min); TLC: Rε = 0.41 (DCM : MeOH (9 : 0.5; *v*/*v*)); C₁₄H₁₈N₂O₂ (246.31), monoisotope mass: 264.13. UPLC (purity: > 99.9%): t_{R} = 3.90 min, (M + H)⁺ 247.2. ¹H NMR (500 MHz, CDCl₃) δ 1.35 (d, *J* = 7.2 Hz, 3H), 1.94-1.99 (m, 2H), 2.27-2.35 (m, 2H) , 3.37-3.41 (m, 2H), 4.39 (dd, *J* = 5.9, 2.2 Hz, 2H), 4.65-4.74 (m, 1H), 6.75 (br. s, 1H), 7.20-7.23 (m, 2H), 7.24-7.26 (m, 1H), 7.27-7.32 (m, 2H). ¹³C NMR (126 MHz, CDCl₃) δ 13.8, 18.1, 31.1, 43.5, 43.8, 50.3, 127.5, 127.6, 128.7, 138.3, 170.6, 175.8.

### Compound (2R,S)-3 (wherein R=H): (2R,S)-N-benzyl-2-(2-oxopyrrolidin-1-yl)propanamide

The compound was obtained analogously to the compound **(2R)-3,** using (*R,S*)-*N*-(1-(benzylamino)-1-oxopropan-2-yl)-4-chlorobutanamide (0.63 g, 2.2 mmol, 1 eq) (**IV**, wherein **R**=H) and NaH (0.106 g, 4.4 mmol, 2 eq). The crude product was purified by column chromatography using DCM : MeOH (9 : 0.5; *v*/*v*) solvent system. After washing with Et₂O, the compound was obtained as a white solid. Yield: 88% (0.48 g); mp 78.1-78.6°C; TLC: Rε = 0.41 (DCM : MeOH (9 : 0.5; *v*/*v*)); C₁₄H₁₈N₂O₂ (246.31), monoisotope mass: 264.13. UPLC (purity: > 99.9%): t_{R} = 3.91 min, (M + H)⁺ 247.2. ¹H NMR (500 MHz, CDCl₃) δ 1.36 (d, *J* = 7.2 Hz, 3H), 1.95-1.99 (m, 2H), 2.28-2.36 (m, 2H) , 3.36-3.43 (m, 2H), 4.38 (dd, *J* = 5.9, 2.2 Hz, 2H), 4.70 (q, *J* = 7.2 Hz, 1H), 6.76 (br. s, 1H), 7.19-7.22 (m, 2H), 7.23-7.25 (m, 1H), 7.27-7.31 (m, 2H). ¹³C NMR (126 MHz, CDCl₃) δ 13.8, 18.1, 31.1, 43.5, 43.8, 50.3, 127.5, 127.6, 128.7, 138.3, 170.6, 175.8.

### Compound (2R)-4 (wherein R=F): (2R)-N-(2-fluorobenzyl)-2-(2-oxopyrrolidin-1-yl)propanamide

The compound was obtained analogously to the compound **(2R)-3,** using (*R*)-4-chloro-*N*-(1-((2-fluorobenzyl)amino)-1-oxopropan-2-yl)butanamide (0,57 g, 1.9 mmol, 1 eq) (**IV**, wherein **R**=F) and NaH (0.091 g, 3.8 mmol, 2 eq). The crude product was purified by column chromatography using DCM : MeOH (9 : 0.5; *v*/*v*) solvent system. The compound was obtained as a white solid. Yield: 88% (0.44 g); mp 92.5-93.1°C; Chiral HPLC> 99% ee (t_{R} = 8.959 min); TLC: R_{f} = 0.39 (DCM : MeOH (9 : 0.5; *v*/*v*)); C14H₁₇FN₂O₂ (264.30), monoisotope mass: 264.13. UPLC (purity: > 99.9%): t_{R} = 4.04 min, (M + H)⁺ 265.9. ¹H NMR (500 MHz, CDCl₃) δ 1.34 (d, *J =* 7.2 Hz, 3H), 1.96-2.00 (m, 1H), 2.27-2.44 (m, 2H), 3.30-3.34 (m, 1H), 3.39-3.42 (m, 1H), 4.39 (dd, *J* = 14.9, 5.7 Hz, 1H), 4.47 (dd, *J* = 15.0, 6.2 Hz, 1H), 4.64-4.75 (m, 1H), 4.69 (d, *J* = 7.2 Hz, 1H), 6.69 (br. s, 1H), 7.01 (t, *J* = 9.1 Hz, 1H), 7.07 (t, *J* = 7.4 Hz, 1H), 7.21-7.27 (m, 2H). ¹³C NMR (126 MHz, CDCl₃) δ 13.7, 18.1, 31.1, 37.6, 37.7, 43.7, 50.3, 115.4, 115.5, 124.3, 124.3, 125.1, 125.3, 129.3, 129.4, 130.0, 130.1, 160.0, 162.0, 170.6, 175.8.

### Compound (2S)-4 (wherein R=F): (2S)-N-(2-fluorobenzyl)-2-(2-oxopyrrolidin-1-yl)propanamide

The compound was obtained analogously to the compound **(2*R*)-3,** using (*S*)-4-chloro-*N*-(1-((2-fluorobenzyl)amino)-1-oxopropan-2-yl)butanamide (0.57 g, 1.9 mmol, 1 eq) (**IV**, wherein **R**=F) and NaH (0.091 g, 3.8 mmol, 2 eq). The crude product was purified by column chromatography using DCM : MeOH (9 : 0.5; *v*/*v*) solvent system. The compound was obtained as a white solid. Yield: 78% (0.39 g); mp 92.1-93.8°C; Chiral HPLC> 99% ee (t_{R} = 13.313 min); TLC: R_{f} = 0.40 (DCM : MeOH (9 : 0.5; *v*/*v*)); C₁₄H₁₇FN₂O₂ (264.30), monoisotope mass: 264.13. UPLC (purity: > 99.9%): t_{R} = 4.03 min, (M + H)⁺ 265.9. ¹H NMR (500 MHz, CDCl₃) δ 1.35 (d, *J* = 7.2 Hz, 3H), 1.95-2.00 (m, 1H), 2.26-2.45 (m, 2H) , 3.31-3.36 (m, 1H), 3.38-3.43 (m, 1H), 4.40 (dd, *J* = 14.9, 5.7 Hz, 1H), 4.46 (dd, *J* = 15.0, 6.2 Hz, 1H), 4.64-4.75 (m, 1H), 4.69 (d, *J* = 7.2 Hz, 1H), 6.68 (br. s, 1H), 7.02 (t, *J* = 9.1 Hz, 1H), 7.06 (t, *J* = 7.4 Hz, 1H), 7.21-7.27 (m, 2H). ¹³C NMR (126 MHz, CDCl₃) δ 13.7, 18.1, 31.1, 37.6, 37.7, 43.7, 50.3, 115.4, 115.5, 124.3, 124.3, 125.1, 125.3, 129.3, 129.4, 130.0, 130.1, 160.0, 162.0, 170.6, 175.8.

**Zwi** 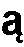 **zek (2*R*,*S*)-4** (wherein **R**=F): (*2R,S*)*-N-(2-fluorobenzyl)-2-(2-oxopyrrolidin-1-yl)propanamide*

The compound was obtained analogously to the compound **(2R)-3,** using (R,S)-4-chloro-N-(1-((2-fluorobenzyl)amino)-1-oxopropan-2-yl)butanamide (0.57 g, 1.9 mmol, 1 eq) (**IV**, wherein **R**=F) and NaH (0.091 g, 3.8 mmol, 2 eq). The crude product was purified by column chromatography using DCM : MeOH (9 : 0.5; *v*/*v*) solvent system. The compound was obtained as a white solid. Yield: 82% (0.41 g); mp 75.4-76.9°C; TLC: R_{f} = 0.41 (DCM : MeOH (9 : 0.5; *v*/*v*)); C₁₄H₁₇FN₂O₂ (264.30), monoisotope mass: 264.13. UPLC (purity: > 99.9%): t_{R} = 4.03 min, (M + H)⁺ 265.9. ¹H NMR (500 MHz, CDCl₃) δ 1.34 (d, *J* = 7.2 Hz, 3H), 1.94-2.01 (m, 1H), 2.24-2.45 (m, 2H) , 3.30-3.35 (m, 1H), 3.38-3.44 (m, 1H), 4.39 (dd, *J =* 14.9, 5.7 Hz, 1H), 4.47 (dd, *J* = 15.0, 6.2 Hz, 1H), 4.63-4.76 (m, 1H), 4.70 (d, *J* = 7.2 Hz, 1H), 6.69 (br. s, 1H), 7.01 (t, *J =* 9.1 Hz, 1H), 7.06 (t, *J* = 7.4 Hz, 1H), 7.19-7.28 (m, 2H). ¹³C NMR (126 MHz, CDCl₃) δ 13.7, 18.1, 31.1, 37.6, 37.7, 43.7, 50.3, 115.4, 115.5, 124.3, 124.3, 125.1, 125.3, 129.3, 129.4, 130.1, 130.2, 160.0, 162.1, 170.6, 175.8.

### Examples of synthesis, physicochemical and spectral data of intermediates V and Vi, according to Scheme 2:

### Intermediate V (wherein R=H) (R)-4-((1-(benzylamino)-1-oxopropan-2-yl)amino)-4-oxobut-2-ene acid

Maleic anhydride (0.33 g, 3.4 mmol, 1 eq) was added to a solution of (*R*)-2-amino-*N*-benzylpropanamide (0.6 g, 3.4 mmol, 1 eq) (**III**) in AcOEt (40 mL), and the mixture was stirred for 30 min. Next, AcOEt was evaparated to dryness. After washing with Et₂O, the compound was obtained as a white solid. Yield: 98% (0.91 g); TLC: R_{f} = 0.28 (DCM : MeOH (9 : 0.1; *v*/*v*)); C₁₄H₁₆N₂O₄ (276.29), monoisotopic mass: 276.11. UPLC (> 99.9% purity): t_{R} = 3.72 min. (M + H)⁺ 278.2.

### Intermediate VI (wherein R=H) (R)-N-benzyl-2-(2,5-dioxo-2,5-dihydro-1H-pyrrol-1-yl)propanamide

ZnCl₂ (0.39 g, 10 mmol, 1 eq) was added to a suspension of (*R*)-4-((1-(benzylamino)-1-oxopropan-2-yl)amino)-4-oxobut-2-ene acid (0.80 g, 2.9 mmol, 1 eq) (**V**, wherein **R**=H) in dry benzene (20 mL), and the mixture was stirred, and heated to 80°C. Next, a solution of HMDS (0.70 g, 0.91 mL, 4.35 mmol, 1.5 eq) in dry benzene (8 mL) was added dropwise, during 30 minutes. The reaction was stirred at reflux for 24 hours, and next was concentrated under reduced pressure. The oily residue was dissolved in DCM, and washed with 0.1 M HCl (3 × 50 mL), water (3 × 50 mL), and brine (3 × 50 mL). The organic layer was dried over Na₂SO₄, and evaporated to dryness. The crude product was purified by column chromatography using DCM : MeOH (9 : 0.5; *v*/*v*) solvent system. After washing with Et₂O, the compound was obtained as a white solid. Yield: 80% (0.60 g); mp 98.3-98.8°C; Chiral HPLC > 99% ee (t_{R} = 50.631 min); TLC: R_{f} = 0.34 (DCM : MeOH (9 : 0.5; *v*/*v*)); C₁₄H₁₄N₂O₃ (258.28), monoisotopic mass: 258.10. UPLC (purity 100%): t_{R} = 4.41 min. (M + H)⁺ 259.2.

### Intermediate VI (wherein R=H) (S)-N-benzyl-2-(2,5-dioxo-2,5-dihydro-1H-pyrrol-1-yl)propanamide

The compound was obtained analogously to the compound (***R***)-**VI** (wherein **R**=H) using (*S*)-4-((1-(benzylamino)-1-oxopropan-2-yl)amino)-4-oxobut-2-ene acid (0.80 g, 2.9 mmol, 1 eq) (**V**, wherein **R**=H), ZnCl₂ (0.39 g, 10 mmol, 1 eq) and HMDS (0.70 g, 0.91 ml, 4.35 mmol, 1.5 eq). The crude product was purified by column chromatography using DCM : MeOH (9 : 0.5; *v*/*v*) solvent system. After washing with Et₂O, the compound was obtained as a white solid. Yield: 83% (0.62 g); mp 97.9-98.6°C; Chiral HPLC > 99% ee (t_{R} = 52.970 min); TLC: Rε = 0.34 (DCM : MeOH (9 : 0.5; *v*/*v*)); C₁₄H₁₄N₂O₃ (258.28), monoisotopic mass: 258.10. UPLC (purity 100%): t_{R} = 4.40 min. (M + H)⁺259.2.

### Intermediate VI (wherein R=H) (R,S)-N-benzyl-2-(2,5-dioxo-2,5-dihydro-1H-pyrrol-1-yl)propanamide

The compound was obtained analogously to the compound (***R***)-**VI** (wherein **R**=H) using (*R,S*)-4-((1-(benzylamino)-1-oxopropan-2-yl)amino)-4-oxobut-2-ene acid (0.80 g, 2.9 mmol, 1 eq) (**V**, wherein **R**=H), ZnCl₂ (0.39 g, 10 mmol, 1 eq) and HMDS (0.70 g, 0.91 mL, 4.35 mmol, 1.5 eq). The crude product was purified by column chromatography using DCM : MeOH (9 : 0.5; *v*/*v*) solvent system. After washing with Et₂O, the compound was obtained as a white solid. Yield: 74% (0.55 g); mp 85.5-86.7°C; TLC: R_{f} = 0.34 (DCM : MeOH (9 : 0.5; *v*/*v*)); C₁₄H₁₄N₂O₃ (258.28), monoisotopic mass: 258.10. UPLC (purity 100%): t_{R} = 4.39 min. (M + H)⁺ 259.2.

### Intermediate VI (wherein R=F) (R)-2-(2,5-dioxo-2,5-dihydro-1H-pyrrol-1 -yl)-N-(2-fluorobenzyl)propanamide

The compound was obtained analogously to the compound (***R***)-**VI** (wherein **R**=H) using (R)-4-((1-((2-fluorobenzyl)amino)-1-oxopropan-2-yl)amino)-4-oxobut-2-ene acid (0.85 g, 2.9 mmol, 1 eq) (**V**, wherein **R**=F), ZnCl₂ (0.39 g, 10 mmol, 1 eq) and HMDS (0.70 g, 0.91 ml, 4.35 mmol, 1.5 eq). The crude product was purified by column chromatography using DCM : MeOH (9 : 0.5; *v*/*v)* solvent system. The compound was obtained as a whitewhite oil. Yield: 82% (0.65 g); Chiral HPLC > 99% ee (t_{R} = 35.607 min); TLC: Rε = 0.75 (DCM : MeOH (9 : 0.5; *v*/*v*)); C₁₄H₁₃FN₂O₃ (276.27), monoisotopic mass: 276.09. UPLC (purity 99.6%): t_{R} = 4.55 min. (M + H)⁺ 277.2.

### Intermediate VI (wherein R=F) (S)-2-(2,5-dioxo-2,5-dihydro-1H-pyrrol-1-yl)-N-(2-fluorobenzyl)propanamide

The compound was obtained analogously to the compound (***R***)-**VI** (wherein **R**=H) using (*S*)-4-((1-((2-fluorobenzyl)amino)-1-oxopropan-2-yl)amino)-4-oxobut-2-ene acid (0.85 g, 2.9 mmol, 1 eq) (**V**, wherein **R**=F), ZnCl₂ (0.39 g, 10 mmol, 1 eq) and HMDS (0.70 g, 0.91 ml, 4.35 mmol, 1.5 eq). The crude product was purified by column chromatography using DCM : MeOH (9 : 0.5; *v*/*v*) solvent system. The compound was obtained as a whitewhite oil. Yield: 84% (0.67 g); Chiral HPLC > 99% ee (t_{R} = 38.845 min); TLC: Rε = 0.75 (DCM : MeOH (9: 0.5; *v*/*v*)); C₁₄H₁₃FN₂O₃ (276.27), monoisotopic mass: 276.09. UPLC (purity 99.5%): t_{R} = 4.55 min. (M + H)⁺ 277.1.

### Intermediate VI (wherein R=F) (R,S)-2-(2,5-dioxo-2,5-dihydro-1H-pyrrol-1-yl)-N-(2-fluorobenzyl)propanamide

The compound was obtained analogously to the compound (***R***)-**VI** (wherein **R**=H) using (R,S)-4-((1-((2-fluorobenzyl)amino)-1-oxopropan-2-yl)amino)-4-oxobut-2-ene acid (0.85 g, 2.9 mmol, 1 eq) (**V**, wherein **R**=F), ZnCl₂ (0.39 g, 10 mmol, 1 eq) and HMDS (0.70 g, 0.91 ml, 4.35 mmol, 1.5 eq). The crude product was purified by column chromatography using DCM : MeOH (9 : 0.5; *v*/*v)* solvent system. The compound was obtained as a whitewhite oil. Yield: 77% (0.61 g); TLC: R_{f} = 0.75 (DCM : MeOH (9 : 0.5; *v*/*v*)); C₁₄H₁₃FN₂O₃ (276.27), monoisotopic mass: 276.09. UPLC (purity 99.8%): t_{R} = 4.56 min. (M + H)⁺ 277.2.

### Synthesis,physicochemical and spectral data of the final compounds (2R)-5, (2S)-5, (2R,S)-5 i (2R)-6, (2S)-6, (2R,S)-6

### Compound (2R)-5: (2R)-N-benzyl-2-(3-(dimethylamino)-2,5-dioxopyrrolidin-1 -yl)propanamide hydrochloride

2 M solution of dimethylamine (0.105 g, 2.3 mmol, 1 eq) in THF was added to a solution of (R)-N-benzyl-2-(2,5-dioxo-2,5-dihydro-1*H*-pyrrol-1-yl)propanamide (0.60 g, 2.3 mmol, 1 eq, **VI** wherein **R**=H) in dry benzene (30 mL). The crude product was purified by column chromatography using DCM : MeOH (9 : 0.5; *v*/*v*) solvent system. Next, the compound was converted into its hydrochloride salt, by treating the compound with 2M methanolic hydrochloric acid. The product was obtained as a whitewhite solid. Yield: 88% (0.62 g); mp 115.8-116.9°C; TLC: R_{f} = 0.36 (DCM : MeOH (9 : 0.5; *v*/*v*)); C₁₆H₂₂ClN₃O₃ (339.82), monoisotope mass: 303.16. UPLC (purity: > 99.9%): t_{R} = 2.79 min, (M + H)⁺ 304.2. ¹H NMR (500 MHz, CDCl₃) δ 1.52 (d, *J* = 7.2 Hz, 3H), 2.78 (s, 6H), 2.85 (br. s, 3H), 4.34 (d , *J* = 6.0 Hz, 2H), 4.74-4.77 (m, 1H), 7.18-7.24 (m, 2H), 7.25-7.29 (m, 3H), 7.59 (br. s, 1H), 7.73 (br. s, 1H). ¹³C NMR (126 MHz, CDCl₃) δ 14.2, 14.2, 31.0, 32.1, 32.2 , 41.8, 42.1, 43.6, 49.9, 50.0, 61.0, 61.3, 127.3, 127.4, 127.6, 127.8, 128.6, 128.6, 138.5, 138.6, 167.9, 168.1, 171.8.

### Compound (2S)-5: (2S)-N-benzyl-2-(3-(dimethylamino)-2,5-dioxopyrrolidin-1-yl)propanamide hydrochloride

The compound was obtained analogously to the compound **(2*R*)-5.** 2 M solution of dimethylamine (0.105 g, 2.3 mmol, 1 eq) in THF was added to a solution of (*S*)*-N*-benzyl-2-(2,5-dioxo-2,5-dihydro-1*H*-pyrrol-1-yl)propanamide (0.60 g, 2.3 mmol, 1 eq, **VI** wherein **R**=H) in dry benzene (30 mL). The crude product was purified by column chromatography using DCM : MeOH (9 : 0.5; *v*/*v*) solvent system. Next, the compound was converted into its hydrochloride salt, by treating the compound with 2M methanolic hydrochloric acid. The product was obtained as a whitewhite solid. Yield: 83% (0.58 g); mp 115.4-116.7°C; TLC: R_{f} = 0.37 (DCM : MeOH (9 : 0.5; *v*/*v*)); C₁₆H₂₂ClN₃O₃ (339.82), monoisotope mass: 303.16. UPLC (purity: > 99.9%): t_{R} = 2.78 min, (M + H)⁺ 304.2. ¹H NMR (500 MHz, CDCl₃) δ 1.53 (d, *J* = 7.2 Hz, 3H), 2.79 (s, 6H), 2.84 (br. s, 3H), 4.35 (d , *J* = 6.0 Hz, 2H), 4.73-4.79 (m, 1H), 7.16-7.21 (m, 2H), 7.27-7.31 (m, 3H), 7.58 (br. s, 1H), 7.74 (br. s, 1H). ¹³C NMR (126 MHz, CDCl₃) δ 14.1, 14.2, 31.2, 32.1, 32.2 , 41.7, 42.1, 43.6, 49.8, 50.0, 61.1, 61.2, 127.3, 127.5, 127.6, 127.9, 128.5, 128.6, 138.5, 138.7, 167.9, 168.2, 171.9.

### Compound (2R,S)-5: (2R,S)-N-benzyl-2-(3-(dimethylamino)-2,5-dioxopyrrolidin-1-yl)propanamide hydrochloride

The compound was obtained analogously to the compound **(2*R*)-5.** 2 M solution of dimethylamine (0.105 g, 2.3 mmol, 1 eq) in THF was added to a solution of (*R,S*)-*N*-benzyl-2-(2,5-dioxo-2,5-dihydro-1*H*-pyrrol-1-yl)propanamide (0.60 g, 2.3 mmol, 1 eq, **VI** wherein **R**=H) in dry benzene (30 mL). The crude product was purified by column chromatography using DCM : MeOH (9 : 0.5; *v*/*v)* solvent system. Next, the compound was converted into its hydrochloride salt, by treating the compound with 2M methanolic hydrochloric acid. The product was obtained as a whitewhite solid. Yield: 86% (0.60 g); mp 97.2-98.5°C; TLC: R_{f} = 0.38 (DCM : MeOH (9 : 0.5; *v*/*v*)); C₁₆H₂₂ClN₃O₃ (339.82), monoisotope mass: 303.16. UPLC (purity: > 99.9%): t_{R} = 2.79 min, (M + H)⁺ 304.2. ¹H NMR (500 MHz, CDCl₃) δ 1.54 (d, *J* = 7.2 Hz, 3H), 2.80 (s, 6H), 2.85 (br. s, 3H), 4.28-4.38 (m, 2H), 4.72-4.78 (m, 1H), 7.15-7.22 (m, 2H), 7.25-7.33 (m, 3H), 7.59 (br. s, 1H), 7.73 (br. s, 1H). ¹³C NMR (126 MHz, CDCl₃) δ 14.2, 14.3, 31.2, 32.0, 32.3, 41.8, 42.1, 43.6, 49.7, 50.1, 61.1, 61.2, 127.4, 127.5, 127.7, 127.9, 128.4, 128.7, 138.4, 138.8, 167.7, 168.3, 171.8.

### Compound (2R)-6: (2R)-2-(3-(dimethylamino)-2,5-dioxopyrrolidin-1-yl)-N-(2-fluorobenzyl)propanamide hydrochloride

The compound was obtained analogously to the compound **(2*R*)-5,** but using (R)-2-(2,5-dioxo-2,5-dihydro-1*H*-pyrrol-1-yl)-*N*-(2-fluorobenzyl)propanamide (0.64 g, 2. mmol, 1 eq, **VI,** wherein **R**=F), and 2 M solution of dimethylamine (0.105 g, 2.3 mmol, 1 eq) in THF. The crude product was purified by column chromatography using DCM : MeOH (9 : 0.5; *v*/*v*) solvent system. Next, the compound was converted into its hydrochloride salt, by treating the compound with 2M methanolic hydrochloric acid. The product was obtained as a whitewhite solid. Yield: 85% (0.63 g); mp 118.4-119.6°C; TLC: R_{f} = 0.45 (DCM : MeOH (9 : 0.5; *v*/*v*)); C₁₆H₂₁ClFN₃O₃ (357.81), monoisotope mass: 321.15. UPLC (purity: 99.9%): t_{R} = 2.91 min, (M + H)⁺ 322.2. ¹H NMR (500 MHz, CDCl₃) δ 1.52 (dd, *J* = 11.5, 7.5 Hz, 3H), 2.94 (s, 6H), 3.08-3.36 (m, 3H) , 4.38-4.44 (m, 2H), 4.76 (dd, *J* = 15.0, 7.30 Hz, 1H), 6.93-7.01 (m, 1H), 7.03 -7.10 (m, 1H), 7.19 (dd, *J* = 4.7, 2.2 Hz, 1H), 7.26-7.33 (m, 1H), 7.61 (br. s, 1H), 7.8 (br. s, 1H). ¹³C NMR (126 MHz, CDCl₃) δ 14.2, 31.0, 32.2, 32.4, 37.5, 50.0, 50.0, 60.9, 61.2, 115.2, 115.3, 115.3, 124.3, 129.1, 129.2, 129.8, 130.0, 159.8, 161.7, 167.9, 168.0, 171.4.

### Compound (25)-6: (2S)-2-(3-(dimethylamino)-2,5-dioxopyrrolidin-1-yl)-N-(2-fluorobenzyl)propanamide hydrochloride

The compound was obtained analogously to the compound **(2*R*)-5,** but using (S)-2-(2,5-dioxo-2,5-dihydro-1*H*-pyrrol-1-yl)-*N*-(2-fluorobenzyl)propanamide (0.64 g, 2. mmol, 1 eq, **VI,** wherein **R**=F), and 2 M solution of dimethylamine (0.105 g, 2.3 mmol, 1 eq) in THF. The crude product was purified by column chromatography using DCM : MeOH (9 : 0.5; *v*/*v*) solvent system. Next, the compound was converted into its hydrochloride salt, by treating the compound with 2M methanolic hydrochloric acid. The product was obtained as a whitewhite solid. Yield: 89% (0.65 g); mp 118.5-119.8°C; TLC: Rε = 0.46 (DCM : MeOH (9 : 0.5; *v*/*v*)); C₁₆H₂₁ClFN₃O₃ (357.81), monoisotope mass: 321.15. UPLC (purity: > 99.9%): t_{R} = 2.92 min, (M + H) + 322.2. ¹H NMR (500 MHz, CDCl₃) δ 1.53 (dd, *J* = 11.5, 7.3 Hz, 3H), 2.93 (s, 6H), 3.07-3.35 (m, 3H) , 4.38-4.43 (m, 2H), 4.77 (dd, *J* = 15.2, 7.5 Hz, 1H), 6.97 (t, *J=* 8.8 Hz, 1H), 7.03-7.11 (m, 1H), 7.19 (dd, *J* = 4.8, 2.3 Hz, 1H), 7.27-7.33 (m, 1H), 7.57 (br. s, 1H), 7.78 (br. s,, 1H). ¹³C NMR (126 MHz, CDCl₃) δ 14.2, 31.0, 32.2, 32.4, 37.5, 50.0, 50.0, 60.9, 61.2, 115.2, 115.3, 115.3, 124.3, 129.1, 129.2, 129.8, 130.0, 159.8, 161.7, 167.9, 168.0, 171.4.

### Compound (2R,S)-6: (2R,S)-2-(3-(dimethylamino)-2,5-dioxopyrrolidin-1-yl)-N-(2-fluorobenzyl)propanamide hydrochloride

The compound was obtained analogously to the compound **(2*R*)-5,** but using (R,S)-2-(2,5-dioxo-2,5-dihydro-1*H*-pyrrol-1-yl)-*N*-(2-fluorobenzyl)propanamide (0.64 g, 2. mmol, 1 eq, **VI,** wherein **R**=F), and 2 M solution of dimethylamine (0.105 g, 2.3 mmol, 1 eq) in THF. The crude product was purified by column chromatography using DCM : MeOH (9 : 0.5; *v*/*v*) solvent system. Next, the compound was converted into its hydrochloride salt, by treating the compound with 2M methanolic hydrochloric acid. The product was obtained as a whitewhite solid. Yield: 75% (0.54 g); mp 104.2-105.7°C; TLC: Rε = 0.47 (DCM : MeOH (9 : 0.5; *v*/*v*)); C₁₆H₂₁ClFN₃O₃ (357.81), monoisotope mass: 321.15. UPLC (purity: > 99.9%): t_{R} = 2.90 min, (M + H)⁺ 322.2. ¹H NMR (500 MHz, CDCl₃) δ 1.53 (dd, *J* = 11.0, 7.5 Hz, 3H), 2.93 (s, 6H), 2.99 (br.s, 3H), 4.38-4.43 (m, 2H), 4.78 (dd, *J =* 15.5, 7.5 Hz, 1H), 6.97 (t, *J =* 8.9 Hz, 1H), 7.06 (t, *J* = 7.5 Hz, 1H), 7.18-7.21 (m, 1H), 7.27-7.33 (m, 1H), 7.57 (br.s, 1H) , 7.76 (br. s,, 1H). ¹³C NMR (126 MHz, CDCl₃) δ 14.2, 14.2, 31.0, 32.2, 32.4, 37.5, 50.0, 50.0, 60.9, 61.2, 115.2, 115.3, 115.3, 124.3, 125.2, 129.1, 129.1, 129.2, 129.8, 130.0, 159.7, 161.7, 167.9, 168.0, 171.3.

### Preparation and physicochemical data of other salts, i.e. sulfate, methanesulfonate, toluenesulfonate, succinate, fumarate or lactate, of compounds (2R)-5, (2S)-5, (2R,S)-5, (2R)-6, (2S)-6 or (2R,S) according to the invention

All salts were prepared following routine synthetic procedure. The procedure consisted of dissolving of 1 eq of compound **(2*R*)-5, (2*S*)-5, (2*R*,*S*)-5, (2*R*)-6, (2*S*)-6** or **(2*R*,*S*)-6** (as a free base) in anhydrous ethanol, and addition of a suitable acid, i.e. 1 eq of methanesulfonic, toluenesulfonic or lactic acid, or 0.5 eq of concentrated sulfuric, succinic or fumaric acid. The mixture was stirred for 15 minutes at room temperature, and the solvent was distilled off under reduced pressure. The residue was washed with diethyl ether, to give the appropriate salt as a white powder.

### Compound (2R)-5: (2R)-N-benzyl-2-(3-(dimethylamino)-2,5-dioxopyrrolidin-1 -yl)propanamide methanesulfonate

White solid; mp 116,4-117,2°C; C₁₇H₂₅N₃O₆S (399.46), monoisotope mass: 303.16. UPLC (purity: > 99.9%): t_{R} = 2.83 min, (M + H)⁺ 304.2. ¹H NMR (500 MHz, CDCl₃) δ 1.57 (dd, *J* = 7.3, 4.2 Hz, 3H), 2.16 (s, 3H), 2.67 (s, 6H), 2.99-3.10 (m, 2H), 4.29-4.38 (m, 1H), 4.38-4.55 (m, 1H), 4.79 (dd, *J* = 15.2, 7,5 Hz, 2H), 7.20-7.24 (m, 3H), 7.25-7.33 (m, 3H), 7.44 (br.s, 1H). ¹³C NMR (126 MHz, CDCl₃) δ 14.1, 14.2, 31.2, 32.1, 32.2, 41.7, 42.1, 43.6, 44.5, 49.8, 50.0, 61.1, 61.2, 127.3, 127.5, 127.6, 127.9, 128.5, 128.6, 138.5, 138.7, 167.9, 168.2, 171.9.

### Compound (2S)-5: (2S)-N-benzyl-2-(3-(dimethylamino)-2,5-dioxopyrrolidin-1-yl)propanamide methanesulfonate

White solid; mp 116,1-116.9°C; C₁₇H₂₅N₃O₆S (399,46), monoisotope mass: 303.16. UPLC (purity: > 99.9%): t_{R} = 2.80 min, (M + H)⁺ 304.2. ¹H NMR (500 MHz, CDCl₃) δ 1.58 (dd, *J* = 7.3, 4.2 Hz, 3H), 2.15 (s, 3H), 2.68 (s, 6H), 2.99-3.12 (m, 2H), 4.30-4.39 (m, 1H), 4.41-4.57 (m, 1H), 4.78 (dd, *J* = 15.2, 7.5 Hz, 2H), 7.20-7.25 (m, 3H), 7.26-7.32 (m, 3H), 7.44 (br. s, 1H). ¹³C NMR (126 MHz, CDCl₃) δ 14.1, 14.2, 31.2, 32.1, 32.2, 41.7, 42.1, 43.6, 44.5, 49.8, 50.0, 61.1, 61.2, 127.3, 127.5, 127.6, 127.9, 128.5, 128.6, 138.5, 138.7, 167.9, 168.2, 171.9.

### Compound (2R,S)-5: (2R,S)-N-benzyl-2-(3-(dimethylamino)-2,5-dioxopyrrolidin-1-yl)propanamide methanesulfonate

White solid; mp 102,2-102,8°C; C₁₇H₂₅N₃O₆S (399.46), monoisotope mass: 303.16. UPLC (purity: > 99.9%): t_{R} = 2.82 min, (M + H)⁺ 304.2. ¹H NMR (500 MHz, CDCl₃) δ 1.56 (dd, *J* = 7.3, 4.2 Hz, 3H), 2.16 (s, 3H), 2.67 (s, 6H), 2.98-3.09 (m, 2H), 4.28-4.39 (m, 1H), 4.40-4.58 (m, 1H), 4.78 (dd, *J* = 15.2, 7.5 Hz, 2H), 7.21-7.25 (m, 3H), 7.26-7.34 (m, 3H), 7.45 (br. s, 1H). ¹³C NMR (126 MHz, CDCl₃) δ 14.1, 14.2, 31.2, 32.1, 32.2, 41.7, 42.1, 43.6, 44.5, 49.8, 50.0, 61.1, 61.2, 127.3, 127.5, 127.6, 127.9, 128.5, 128.6, 138.5, 138.7, 167.9, 168.2, 171.9.

### Compound (2R)-5: (2R)-N-benzyl-2-(3-(dimethylamino)-2,5-dioxopyrrolidin-1-yl)propanamide toluenesulfonate

White solid; mp 116.8-117.4°C; C₂₃H₂₉N₃O₆S (475.56), monoisotope mass: 303.16. UPLC (purity: > 99.9%): t_{R} = 2.82 min, (M + H)⁺ 304.2. ¹H NMR (500 MHz, CDCl₃) δ 1.47-1.50 (m, 3H), 2.34 (s, 3H), 2.87-2.99 (m, 6H), 3.08-3.36 (m, 2H), 4.17-4.26 (m, 2H), 4.57-4.63 (m, 1H), 4.73-4.75 (m, 1H), 7.08-7.15 (m, 4H), 7.17-7.24 (m, 3H), 7.51-7.58 (m, 3H), 7.68 (br. s, 1H). ¹³C NMR (126 MHz, CDCl₃) δ 14.2, 14.2, 31.0, 32.1, 32.2, 21.3, 41.8, 42.1, 43.6, 49.9, 50.0, 61.0, 61.3, 127.3, 127.4, 127.6, 127.8, 128.2, 128.5, 128.6, 130.0, 138.5, 138.6, 139.4, 142.1, 167.9, 168.1, 171.8.

### Compound (2S)-5: (2S)-N-benzyl-2-(3-(dimethylamino)-2,5-dioxopyrrolidin-1-yl)propanamide toluenesulfonate

White solid; mp 116.2-117.1°C; C₂₃H₂₉N₃O₆S (475.56), monoisotope mass: 303.16. UPLC (purity: > 99.9%): t_{R} = 2.82 min, (M + H)⁺ 304.2. ¹H NMR (500 MHz, CDCl₃) δ 1.48-1.51 (m, 3H), 2.34 (s, 3H), 2.87-2.98 (m, 6H), 3.08-3.37 (m, 2H), 4.18-4.27 (m, 2H), 4.57-4.62 (m, 1H), 4.74-4.78 (m, 1H), 7.07- 7.13 (m, 4H), 7.16-7.23 (m, 3H), 7.50-7.58 (m, 3H), 7.64 (br. s, 1H). ¹³C NMR (126 MHz, CDCl₃) δ 14.2, 14.2, 31.0, 32.1, 32.2, 21.3, 41.8, 42.1, 43.6, 49.9, 50.0, 61.0, 61.3, 127.3, 127.4, 127.6, 127.8, 128.2, 128.5, 128.6, 130.0, 138.5, 138.6, 139.4, 142.1, 167.9, 168.1, 171.8.

### Compound (2R,S)-5: (2R,S)-N-benzyl-2-(3-(dimethylamino)-2,5-dioxopyrrolidin-1-yl)propanamide toluenesulfonate

White solid; mp 101.2-101.9°C; C₂₃H₂₉N₃O₆S (475.56), monoisotope mass: 303.16. UPLC (purity: > 99.9%): t_{R} = 2.81 min, (M + H)⁺ 304.2. ¹H NMR (500 MHz, CDCl₃) δ 1.48-1.51 (m, 3H), 2.33 (s, 3H), 2.87-2.97 (m, 6H), 3.07-3.37 (m, 2H), 4.18-4.26 (m, 2H), 4.56-4.61 (m, 1H), 4.74-4.76 (m, 1H), 7.08- 7.13 (m, 4H), 7.16-7.23 (m, 3H), 7.50-7.58 (m, 3H), 7.66 (br. s, 1H). ¹³C NMR (126 MHz, CDCl₃) δ 14.2, 14.2, 31.0, 32.1, 32.2, 21.3, 41.8, 42.1, 43.6, 49.9, 50.0, 61.0, 61.3, 127.3, 127.4, 127.6, 127.8, 128.2, 128.5, 128.6, 130.0, 138.5, 138.6, 139.4, 142.1, 167.9, 168.1, 171.8.

### Compound (2R)-5: (2R)-N-benzyl-2-(3-(dimethylamino)-2,5-dioxopyrrolidin-1-yl)propanamide lactate

White solid; mp 116.2-116.9°C; C₁₉H₂₇N₃O₆ (393.44), monoisotope mass: 303.16. UPLC (purity: > 99.9%): t_{R} = 2.76 min, (M + H)⁺ 304.2. ¹H NMR (500 MHz, CDCl₃) δ 1.40-1.43 (m, 6H), 2.55 (d, *J* = 9.2 Hz, 6H), 2.84 (dd, *J* = 17.3, 5.3 Hz, 1H), 2.90-3.00 (m, 1H), 3.95 (ddd, *J* = 11.7, 8.9, 5.4, Hz, 1H), 4.26 (q, *J* = 7.0 Hz, 2H), 4.40-4.52 (m, 2H), 4.83 (dd, *J* = 11.2, 7.5 Hz, 1H), 5.32 (br. s , 2H), 6.44 (br. s, 1H), 7.26-7.32 (m, 3H), 7.33-7.35 (m, 2H). ¹³C NMR (126 MHz, CDCl₃) δ 14.1, 14.2, 19.4, 31.2, 32.1, 32.2, 41.7, 42.1, 43.6, 49.8, 50.0, 61.1, 61.2, 77.8, 127.3, 127.5, 127.6, 127.9, 128.5, 128.6, 138.5, 138.7, 167.9, 168.2, 171.9, 176.9.

### Compound (2S)-5: (2S)-N-benzyl-2-(3-(dimethylamino)-2,5-dioxopyrrolidin-1-yl)propanamide lactate

White solid; mp 115.7-116.4°C; C₁₉H₂₇N₃O₆ (393.44), monoisotope mass: 303.16. UPLC (purity: > 99.9%): t_{R} = 2.76 min, (M + H)⁺ 304.2. ¹H NMR (500 MHz, CDCl₃) δ 1.41-1.43 (m, 6H), 2.56 (d, *J* = 9.2 Hz, 6H), 2.84 (dd, *J* = 17.3, 5.3 Hz, 1H), 2.91-3.00 (m, 1H), 3.94 (ddd, *J* = 11.7, 8.9, 5.4 Hz, 1H), 4.25 (q, *J* = 7.0 Hz, 2H), 4.39-4.51 (m, 2H), 4.82 (dd, *J* = 11.2, 7.5 Hz, 1H), 5.33 (br. s, 2H), 6.44 (br. s, 1H), 7.26-7.30 (m, 3H), 7.31-7.34 (m, 2H). ¹³C NMR (126 MHz, CDCl₃) δ 14.1, 14.2, 19.4, 31.2, 32.1, 32.2, 41.7, 42.1, 43.6, 49.8, 50.0, 61.1, 61.2, 77.8, 127.3, 127.5, 127.6, 127.9, 128.5, 128.6, 138.5, 138.7, 167.9, 168.2, 171.9, 176.9.

### Compound (2R,S)-5: (2R,S)-N-benzyl-2-(3-(dimethylamino)-2,5-dioxopyrrolidin-1-yl)propanamide lactate

White solid; mp 100.2-101.1°C; C₁₉H₂₇N₃O₆ (393.44), monoisotope mass: 303.16. UPLC (purity: > 99.9%): t_{R} = 2.76 min, (M + H)⁺ 304.2. ¹H NMR (500 MHz, CDCl₃) δ 1.42 (s, 6H), 2.56 (d, *J* = 9.2 Hz, 6H), 2.85 (dd, *J* = 17.3, 5.3 Hz , 1H), 2.91-3.00 (m, 1H), 3.96 (ddd, *J* = 11.7, 8.9, 5.4 Hz, 1H), 4.25 (q, *J =* 7.0 Hz, 2H), 4.40-4.53 (m, 2H), 4.82 (dd, *J* = 11.2, 7.5 Hz, 1H), 5.31 (br. s, 2H) , 6.44 (br. s, 1H), 7.26-7.31 (m, 3H), 7.33-7.36 (m, 2H). ¹³C NMR (126 MHz, CDCl₃) δ 14.1, 14.2, 19.4, 31.2, 32.1, 32.2, 41.7, 42.1, 43.6, 49.8, 50.0, 61.1, 61.2, 77.8, 127.3, 127.5, 127.6, 127.9, 128.5, 128.6, 138.5, 138.7, 167.9, 168.2, 171.9, 176.9.

### Compound (2R)-5: (2R)-N-benzyl-2-(3-(dimethylamino)-2,5-dioxopyrrolidin-1-yl)propanamide sulfate

White solid; mp 117.1-117.9°C; C₃₂H₄₄N₆O₁₀S (704.80), monoisotope mass: 303.16. UPLC (purity: > 99.9%): t_{R} = 2.76 min, (M + H)⁺ 304.2. ¹H NMR (500 MHz, DMSO-d6) δ 1.49 (dd, *J =* 10.0, 7.2 Hz, 3H), 2.71-2.90 (m, 6H), 2.96-3.13 (m, 2H), 3.15-3.31 (m, 1H), 4.26 (ddd, *J* = 11.2, 9.1, 6.0 Hz, 3H), 4.58-4.74 (m, 1H), 7.15-7.24 (m, 3H), 7.25-7.29 (m, 2H), 10.46-10.79 (m, 1H). ¹³C NMR (126 MHz, CDCl₃) δ 14.1, 14.2, 31.0, 32.1, 32.2, 41.8, 42.1, 43.6, 49.9, 50.0, 61.0, 61.2, 127.3, 127.3, 127.5, 127.8, 128.5, 128.6, 138.5, 138.6, 167.9, 168.1, 171.8.

### Compound (2S)-5: (2S)-N-benzyl-2-(3-(dimethylamino)-2,5-dioxopyrrolidin-1-yl)propanamide sulfate

White solid; 116.8-117.5°C; C₃₂H₄₄N₆O₁₀S (704.80), monoisotope mass: 303.16. UPLC (purity: > 99.9%): t_{R} = 2.76 min, (M + H)⁺ 304.2. ¹H NMR (500 MHz, DMSO-d6) δ 1.48 (dd, *J* = 10.0, 7.2 Hz, 3H), 2.70-2.91 (m, 6H), 2.95-3.12 (m, 2H), 3.14-3.30 (m, 1H), 4.25 (ddd, *J* = 11.2, 9.1, 6.0 Hz, 3H), 4.58-4.74 (m, 1H), 7.13-7.23 (m, 3H), 7.24-7.28 (m, 2H), 10.46-10.80 (m, 1H). ¹³C NMR (126 MHz, CDCl₃) δ 14.1, 14.2, 31.0, 32.1, 32.2, 41.8, 42.1, 43.6, 49.9, 50.0, 61.0, 61.2, 127.3, 127.3, 127.5, 127.8, 128.5, 128.6, 138.5, 138.6, 167.9, 168.1, 171.8.

### Compound (2R,S)-5: (2R,S)-N-benzyl-2-(3-(dimethylamino)-2,5-dioxopyrrolidin-1-yl)propanamide sulfate

White solid; mp 102.7-103.5°C; C₃₂H₄₄N₆O₁₀S (704.80), monoisotope mass: 303.16. UPLC (purity: > 99.9%): t_{R} = 2.76 min, (M + H)⁺ 304.2. ¹H NMR (500 MHz, DMSO-d6) δ 1.49 (dd, *J =* 10.0, 7.2 Hz, 3H), 2.71-2.90 (m, 6H), 2.96-3.13 (m, 2H), 3.15-3.31 (m, 1H), 4.26 (ddd, *J* = 11.2, 9.1, 6.0 Hz, 3H), 4.58-4.74 (m, 1H), 7.15-7.24 (m, 3H), 7.25-7.29 (m, 2H), 10.46-10.79 (m, 1H) ¹³C NMR (126 MHz, CDCl₃) δ 14.1, 14.2, 31.0, 32.1, 32.2, 41.8, 42.1, 43.6, 49.9, 50.0, 61.0, 61.2, 127.3, 127.3, 127.5, 127.8, 128.5, 128.6, 138.5, 138.6, 167.9, 168.1, 171.8.

### Compound (2R)-5: (2R)-N-benzyl-2-(3-(dimethylamino)-2,5-dioxopyrrolidin-1 -yl)propanamide succinate

White solid; mp 116.8-117.5°C; C₃₆H₄₈N₆O₁₀ (724.81), monoisotope mass: 303.16. UPLC (purity: >99.9%): t_{R} = 2.82 min, (M + H)⁺ 304.2. ¹H NMR (500 MHz, CDCl₃) δ 1.48-1.50 (m, 3H), 2.33-2.46 (m, 2H), 2.50-2.68 (m, 2H), 2.98 (s, 6H), 3.10-3.38 (m, 2H), 4.15-4.26 (m, 2H), 4.57-4.61 (m, 1H), 4.72- 4.75 (m, 1H), 7.18-7.29 (m, 3H), 7.49-7.62 (m, 2H), 7.78 (br. s, 1H). ¹³C NMR (126 MHz, CDCl₃) δ 14.2, 14.2, 26.1 31.0, 32.1, 32.2, 39.6, 41.8, 42.1, 43.6, 49.9, 50.0, 56.4, 61.0, 61.3, 127.3, 127.4, 127.6, 127.8, 128.5, 128.6, 138.5, 138.6, 167.9, 168.1, 171.8.

### Compound (2S)-5: (2S)-N-benzyl-2-(3-(dimethylamino)-2,5-dioxopyrrolidin-1-yl)propanamide succinate

White solid; mp 115.9-116.6°C; C₃₆H₄₈N₆O₁₀ (724.81), monoisotope mass: 303.16. UPLC (purity: > 99.9%): t_{R} = 2.82 min, (M + H)⁺ 304.2. ¹H NMR (500 MHz, CDCl₃) δ 1.47-1.50 (m, 3H), 2.32-2.47 (m, 2H), 2.51-2.68 (m, 2H), 2.97 (s, 6H), 3.10-3.38 (m, 2H), 4.15-4.26 (m, 2H), 4.56-4.62 (m, 1H), 4.71-4.74 (m, 1H), 7.19-7.27 (m, 3H), 7.48-7.63 (m, 2H), 7.79 (br. s, 1H). ¹³C NMR (126 MHz, CDCl₃) δ 14.2, 14.2, 26.1 31.0, 32.1, 32.2, 39.6, 41.8, 42.1, 43.6, 49.9, 50.0, 56.4, 61.0, 61.3, 127.3, 127.4, 127.6, 127.8, 128.5, 128.6, 138.5, 138.6, 167.9, 168.1, 171.8.

### Compound (2R,S)-5: (2R,S)-N-benzyl-2-(3-(dimethylamino)-2,5-dioxopyrrolidin-1-yl)propanamide succinate

White solid; mp 101.8-102.5°C; C₃₆H₄₈N₆O₁₀ (724.81), monoisotope mass: 303.16. UPLC (purity: > 99.9%): t_{R} = 2.82 min, (M + H)⁺ 304.2. ¹H NMR (500 MHz, CDCl₃) δ 1.47-1.51 (m, 3H), 2.32-2.45 (m, 2H), 2.51-2.68 (m, 2H), 2.97 (s, 6H), 3.11-3.37 (m, 2H), 4.16-4.25 (m, 2H), 4.56-4.63 (m, 1H), 4.71- 4.78 (m, 1H), 7.20-7.31 (m, 3H), 7.49-7.63 (m, 2H), 7.77 (br. s, 1H). ¹³C NMR (126 MHz, CDCl₃) δ 14.2, 14.3, 26.1 31.0, 32.1, 32.2, 39.6, 41.8, 42.1, 43.6, 49.9, 50.0, 56.4, 61.0, 61.3, 127.3, 127.4, 127.6, 127.8, 128.5, 128.6, 138.5, 138.6, 167.9, 168.1, 171.8.

### Compound (2R)-5: (2R)-N-benzyl-2-(3-(dimethylamino)-2,5-dioxopyrrolidin-1 -yl)propanamide fumarate

White solid; mp 116.4-117.1°C; C₃₆H₄₆N₆O₁₀ (722.80), monoisotope mass: 303.16. UPLC (purity: >99.9%): t_{R} = 2.81 min, (M + H)⁺ 304.2. ¹H NMR (500 MHz, CDCl₃) δ 1.48-1.51 (m, 3H), 2.97 (s, 6H), 3.10-3.39 (m, 2H), 4.14-4.27 (m, 2H), 4.56-4.63 (m, 1H), 4.71-4.74 (m, 1H), 7.19-7.30 (m, 3H), 7.48- 7.65 (m, 4H), 7.89 (br. s, 1H). ¹³C NMR (126 MHz, CDCl₃) δ 14.1, 14.2, 31.0, 32.1, 32.2, 41.8, 42.1, 43.6, 49.9, 50.0, 61.0, 61.2, 127.2, 127.3, 127.5, 127.8, 128.5, 128.6, 133.3, 138.5, 138.6, 167.4, 167.9, 168.1, 171.8.

### Compound (2S)-5: (2S)-N-benzyl-2-(3-(dimethylamino)-2,5-dioxopyrrolidin-1-yl)propanamide fumarate

White solid; mp 115.9-116.6°C; C₃₆H₄₆N₆O₁₀ (722.80), monoisotope mass: 303.16. UPLC (purity: > 99.9%): t_{R} = 2.81 min, (M + H)⁺ 304.2. ¹H NMR (500 MHz, CDCl₃) δ 1.47-1.50 (m, 3H), 2.96 (s, 6H), 3.11-3.38 (m, 2H), 4.15-4,28 (m, 2H), 4.56-4.64 (m, 1H), 4.70-4.72 (m, 1H), 7.18-7.31 (m, 3H), 7.47- 7.64 (m, 4H), 7.87 (br. s, 1H). ¹³C NMR (126 MHz, CDCl₃) δ 14.1, 14.2, 31.0, 32.1, 32.2, 41.8, 42.1, 43.6, 49.9, 50.0, 61.0, 61.2, 127.2, 127.3, 127.5, 127.8, 128.5, 128.6, 133.3, 138.5, 138.6, 167.4, 167.9, 168.1, 171.8.

### Compound (2R,S)-5: (2R,S)-N-benzyl-2-(3-(dimethylamino)-2,5-dioxopyrrolidin-1-yl)propanamide fumarate

White solid; mp 102.1-102.8°C; C₃₆H₄₆N₆O₁₀ (722.80), monoisotope mass: 303.16. UPLC (purity: > 99.9%): t_{R} = 2.81 min, (M + H)⁺ 304.2. ¹H NMR (500 MHz, CDCl₃) δ 1.47-1.51 (m, 3H), 2.96 (s, 6H), 3.10-3.38 (m, 2H), 4.12-4.26 (m, 2H), 4.56-4.62 (m, 1H), 4.70-4.73 (m, 1H), 7.18-7.31 (m, 3H), 7.48- 7.66 (m, 4H), 7.88 (br. s, 1H). ¹³C NMR (126 MHz, CDCl₃) δ 14.1, 14.2, 31.0, 32.1, 32.2, 41.8, 42.1, 43.6, 49.9, 50.0, 61.0, 61.2, 127.2, 127.3, 127.5, 127.8, 128.5, 128.6, 133.3, 138.5, 138.6, 167.4, 167.9, 168.1, 171.8.

### Compound (2R)-6: (2R)-2-(3-(dimethylamino)-2,5-dioxopyrrolidin-1-yl)-N-(2-fluorobenzyl)propanamide methanesulfonate

White solid: mp 119.1-119.9°C; C₁₇H₂₄FN₃O₆S (417.45), monoisotope mass: 321.15. UPLC (purity: > 99.9%): t_{R} = 2.87 min, (M + H)⁺ 322.2. ¹H NMR (500 MHz, CDCl₃) δ 1.53 (dd, *J* = 11.5, 7.5 Hz, 3H), 2.20 (s, 3H), 2.94 (s, 6H), 3.08-3.36 (m, 2H), 4.38-4.44 (m, 3H), 4.76 (dd, *J* = 15.0, 7.3 Hz, 1H), 6.91-7.02 (m, 1H), 7.03-7.10 (m, 1H), 7.19-7.33 (m, 2H), 7.61 (br. s, 1H), 7.89 (br. s, 1H). ¹³C NMR (126 MHz, CDCl₃) δ 16.2, 31.0, 32.3, 32.5, 37.6, 44.5, 50.0, 50.1, 60.9, 61.2, 115.2, 115.3, 115.4, 124.3, 129.1, 129.2, 129.8, 130.0, 159.8, 161.7, 167.9, 168.0, 171.4.

### Compound (2S)-6: (2S)-2-(3-(dimethylamino)-2,5-dioxopyrrolidin-1-yl)-N-(2-fluorobenzyl)propanamide methanesulfonate

White solid: mp 118.7-119.4°C; C₁₇H₂₄FN₃O₆S (417.45), monoisotope mass: 321.15. UPLC (purity: > 99.9%): t_{R} = 2.87 min, (M + H)⁺ 322.2. ¹H NMR (500 MHz, CDCl₃) δ 1.52 (dd, *J* = 11.5, 7.5 Hz, 3H), 2.23 (s, 3H), 2.95 (s, 6H), 3.07-3.37 (m, 2H), 4.39-4.47 (m, 3H), 4.77 (dd, *J* = 15.0, 7.3 Hz, 1H), 6.90-7.04 (m, 1H), 7.05-7.13 (m, 1H), 7.19-7.34 (m, 2H), 7.61 (br. s, 1H), 7.92 (br. s, 1H). ¹³C NMR (126 MHz, CDCl₃) δ 16.1, 31.0, 32.3, 32.5, 37.6, 44.5, 50.0, 50.1, 60.9, 61.2, 115.2, 115.3, 115.4, 124.3, 129.1, 129.3, 129.8, 130.0, 159.8, 161.7, 167.9, 168.0, 171.4.

### Compound (2R,S)-6: (2R,S)-2-(3-(dimethylamino)-2,5-dioxopyrrolidin-1-yl)-N-(2-fluorobenzyl)propanamide methanesulfonate

White solid: mp 105.3-106.1°C; C₁₇H₂₄FN₃O₆S (417.45), monoisotope mass: 321.15. UPLC (purity: > 99.9%): t_{R} = 2.87 min, (M + H) + 322.2. ¹H NMR (500 MHz, CDCl₃) δ 1.54 (dd, *J* = 11.5, 7.50 Hz, 3H), 2.21 (s, 3H), 2.96 (s, 6H), 3.06-3.34 (m, 2H), 4.38-4.46 (m, 3H), 4.78 (dd, *J* = 15.0, 7.3 Hz, 1H), 6.91-7.01 (m, 1H), 7.03-7.11 (m, 1H), 7.19-7.36 (m, 2H), 7.60 (br. s, 1H), 7.94 (br. s, 1H). ¹³C NMR (126 MHz, CDCl₃) δ 16.1, 31.0, 32.3, 32.5, 37.6, 44.5, 50.0, 50.1, 60.9, 61.3, 115.2, 115.3, 115.4, 124.3, 129.2, 129.3, 129.8, 130.0, 159.8, 161.7, 167.9, 168.0, 171.5.

### Compound (2R)-6: (2R)-2-(3-(dimethylamino)-2,5-dioxopyrrolidin-1-yl)-N-(2-fluorobenzyl)propanamide toluenesulfonate

White solid: mp 120.2-120.9°C; C₂₃H₂₈FN₃O₆S (493.55), monoisotope mass: 321.15. UPLC (purity: > 99.9%): t_{R} = 2.93 min, (M + H)⁺ 322.2. ¹H NMR (500 MHz, CDCl₃) δ 1.52 (dd, *J* = 11.5, 7.5 Hz, 3H), 2.34 (s, 3H), 2.94 (s, 6H), 3.08-3.36 (m, 2H), 4.38-4.44 (m, 2H), 4.76 (dd, *J* = 15.0, 7.3 Hz, 1H), 6.93-7.01 (m, 1H), 7.03-7.10 (m, 1H), 7.12-7.21 (m, 4H), 7.26-7.48 (m, 4H), 7.87 (br. s, 1H). ¹³C NMR (126 MHz, CDCl₃) δ 15.2, 21.3, 31.0, 32.2, 32.4, 37.5, 50.0, 50.1, 60.9, 61.2, 115.2, 115.3, 115.4, 124.3, 128.3, 129.1, 129.2, 129.8, 130.0, 131.2, 143.1, 159.8, 161.7 , 167.9, 168.0, 171.4.

### Compound (2S)-6: (2S)-2-(3-(dimethylamino)-2,5-dioxopyrrolidin-1-yl)-N-(2-fluorobenzyl)propanamide toluenesulfonate

White solid: mp 119.5-120.3°C; C₂₃H₂₈FN₃O₆S (493.55), monoisotope mass: 321.15. UPLC (purity: > 99.9%): t_{R} = 2.91 min, (M + H)⁺ 322.2. ¹H NMR (500 MHz, CDCl₃) δ 1.53 (dd, *J* = 11.5, 7.5 Hz, 3H), 2.33 (s, 3H), 2.94 (s, 6H), 3.08-3.37 (m, 2H), 4.37-4.49 (m, 2H), 4.77 (dd, *J* = 15.0, 7.3 Hz, 1H), 6.90-7.02 (m, 1H), 7.04-7.11 (m, 1H), 7.14-7.24 (m, 4H), 7.26-7.47 (m, 4H), 7.88 (br. s, 1H). ¹³C NMR (126 MHz, CDCl₃) δ 15.2, 21.4, 31.0, 32.2, 32.4, 37.5, 50.0, 50.1, 60.8, 61.2, 115.1, 115.3, 115.4, 124.2, 128.3, 129.1, 129.2, 129.8, 130.0, 131.2, 143.1, 159.8, 161.7, 167.9, 168.0, 171.4.

### Compound (2R,S)-6: (2R,S)-2-(3-(dimethylamino)-2,5-dioxopyrrolidin-1-yl)-N-(2-fluorobenzyl)propanamide toluenesulfonate

White solid: mp 106.7-107.5°C; C₂₃H₂₈FN₃O₆S (493.55), monoisotope mass: 321.15. UPLC (purity: > 99.9%): t_{R} =2.93 min, (M + H)⁺ 322.2. ¹H NMR (500 MHz, CDCl₃) δ 1.53 (dd, *J* = 11.5, 7.5 Hz, 3H), 2.33 (s, 3H), 2.94 (s, 6H), 3.08-3.37 (m, 2H), 4.37-4.49 (m, 2H), 4.77 (dd, *J* = 15.0, 7.3 Hz, 1H), 6.90-7.02 (m, 1H), 7.04-7.11 (m, 1H), 7.14-7.24 (m, 4H), 7.26-7.47 (m, 4H), 7.88 (br. s, 1H). ¹³C NMR (126 MHz, CDCl₃) δ 15.2, 21.4, 31.0, 32.2, 32.4, 37.5, 50.0, 50.1, 60.8, 61.2, 115.1, 115.3, 115.4, 124.2, 128.3, 129.1, 129.2, 129.8, 130.0, 131.2, 143.1, 159.8, 161.7, 167.9, 168.0, 171.4.

### Compound (2R)-6: (2R)-2-(3-(dimethylamino)-2,5-dioxopyrrolidin-1-yl)-N-(2-fluorobenzyl)propanamidelactate

White solid: mp 118.8-119.4°C; C₁₉H₂₆FN₃O₆ (411.43), monoisotope mass: 321.15. UPLC (purity: > 99.9%): t_{R} = 2.89 min, (M + H)⁺ 322.2. ¹H NMR (500 MHz, CDCl₃) δ 1.42-1.45 (m, 3H), 2.89 (s, 6H), 3.04-3.36 (m, 3H), 4.38-4.44 (m, 2H), 4.82 (dd, *J* = 12.0, 7.5 Hz, 1H), 5.38 (br.s, 1H), 6.91-7.01 (m, 1H) , 7.03-7.11 (m, 1H), 7.19-7.36 (m, 2H), 7.38-7.46 (m, 2H), 8.05 (br. s, 1H). ¹³C NMR (126 MHz, CDCl₃) δ 14.2, 19.5, 31.0, 32.2, 32.4, 37.5, 50.0, 50.1, 60.9, 61.2, 77.9, 115.2, 115.3, 115.4, 124.3, 129.1, 129.2, 129.8, 130.0, 159.8, 161.7, 167.9, 168.0, 171.4, 178.7.

### Compound (2S)-6: (2S)-2-(3-(dimethylamino)-2,5-dioxopyrrolidin-1-yl)-N-(2-fluorobenzyl)propanamide lactate

White solid: mp 118.1-118.8°C; C₁₉H₂₆FN₃O₆ (411.43), monoisotope mass: 321.15. UPLC (purity: > 99.9%): t_{R} = 2.90 min, (M + H)⁺ 322.2. ¹H NMR (500 MHz, CDCl₃) δ 1.41-1.44 (m, 3H), 2.88 (s, 6H), 3.02-3.37 (m, 3H), 4.38-4.44 (m, 2H), 4.82 (dd, *J* = 12.0, 7.5 Hz, 1H), 5.40 (br. s, 1H), 6.89-7.13 (m, 2H) , 7.18-7.35 (m, 2H), 7.38-7.49 (m, 2H), 8.07 (br. s, 1H). ¹³C NMR (126 MHz, CDCl₃) δ 14.3, 19.5, 31.0, 32.1, 32.4, 37.5, 50.0, 50.1, 60.9, 61.2, 77.9, 115.2, 115.3, 115.4, 124.3, 129.1, 129.2, 129.8, 130.0, 159.8, 161.7, 167.9, 168.0, 171.4, 178.7.

### Compound (2R,S)-6: (2R,S)-2-(3-(dimethylamino)-2,5-dioxopyrrolidin-1-yl)-N-(2-fluorobenzyl)propanamidelactate

White solid: mp 104.9-105.6°C; C₁₉H₂₆FN₃O₆ (411.43), monoisotope mass: 321.15. UPLC (purity: > 99.9%): t_{R} = 2.87 min, (M + H)⁺ 322.2. ¹H NMR (500 MHz, CDCl₃) δ 1.41-1.44 (m, 3H), 2.87 (s, 6H), 3.04-3.35 (m, 3H), 4.37-4.48 (m, 2H), 4.83 (dd, *J* = 12.0, 7.5 Hz, 1H), 5.41 (br. s, 1H), 6.85-7.14 (m, 2H) , 7.19-7.39 (m, 2H), 7.42-7.51 (m, 2H), 8.08 (br. s, 1H). ¹³C NMR (126 MHz, CDCl₃) δ 14.3, 19.5, 31.0, 32.1, 32.4, 37.6, 50.0, 50.1, 60.9, 61.2, 77.9, 115.2, 115.3, 115.4, 124.3, 129.1, 129.3, 129.8, 130.0, 159.8, 161.7, 167.9, 168.0, 171.4, 178.7.

### Compound (2R)-6: (2R)-2-(3-(dimethylamino)-2,5-dioxopyrrolidin-1-yl)-N-(2-fluorobenzyl)propanamide sulfate

White solid: mp 119.4-120.2°C; C₃₂H₄₂F₂N₆O₁₀S (740.78), monoisotope mass: 321.15. UPLC (purity: > 99.9%): t_{R} = 2.89 min, (M + H)⁺ 322.2. ¹H NMR (500 MHz, DMSO-d6) δ 1.46 (dd, *J* = 12.6, 7.2 Hz, 3H), 2.64 (s, 6H), 2.90-3.00 (m, 2H), 3.06 (td, *J* = 17.8, 5.0 Hz, 1H), 4.27 (t, *J* = 5.2 Hz, 2H), 4.39-4.47 (m, 1H), 4.65 (dd, *J* = 7.3, 2.7 Hz, 1H), 7.09-7.15 (m, 2H), 7.21-7.31 (m, 2H), 8.42-8.47 (m, 1H). ¹³C NMR (126 MHz, CDCl₃) δ 14.2, 31.0, 32.2, 32.4, 37.5, 50.0, 50.1, 60.9, 61.2, 115.2, 115.3, 115.4, 124.3, 129.1, 129.2, 129.8, 130.0, 159.8, 161.7, 167.9, 168.0, 171.4.

### Compound (2S)-6: (2S)-2-(3-(dimethylamino)-2,5-dioxopyrrolidin-1-yl)-N-(2-fluorobenzyl)propanamide sulfate

White solid: mp 119.1-119.8°C; C₃₂H₄₂F₂N₆O₁₀S (740.78), monoisotope mass: 321.15. UPLC (purity: > 99.9%): t_{R} = 2.90 min, (M + H)⁺ 322.2. ¹H NMR (500 MHz, DMSO-d6) δ 1.48 (dd, *J* = 12.6, 7.2 Hz, 3H), 2.65 (s, 6H), 2.91-3.03 (m, 2H), 3.06-3.13 (m, 1H), 4.26 (t, *J* = 5.2 Hz, 2H), 4.38-4.46 (m, 1H), 4.64 (dd, *J* = 7.3, 2.7 Hz, 1H), 7.08-7.16 (m, 2H), 7.21-7.30 (m, 2H), 8.42-8.45 (m, 1H). ¹³C NMR (126 MHz, CDCl₃) δ 14.2, 31.0, 32.2, 32.4, 37.5, 50.0, 50.1, 60.9, 61.2, 115.2, 115.3, 115.4, 124.3, 129.1, 129.2, 129.8, 130.0, 159.8, 161.7, 167.9, 168.0, 171.4.

### Compound (2R,S)-6: (2R,S)-2-(3-(dimethylamino)-2,5-dioxopyrrolidin-1-yl)-N-(2-fluorobenzyl)propanamidesulfate

White solid: mp 104.9-105.8°C; C₃₂H₄₂F₂N₆O₁₀S (740.78), monoisotope mass: 321.15. UPLC (purity: > 99.9%): t_{R} = 2.91 min, (M + H)⁺ 322.2. ¹H NMR (500 MHz, DMSO-d6) δ 1.45 (dd, *J* = 12.6, 7.2 Hz, 3H), 2.64 (s, 6H), 2.90-3.00 (m, 2H), 3.06 (td, *J* = 17.8, 5.0 Hz, 1H), 4.27 (t, *J* = 5.2 Hz, 2H), 4.38-4.47 (m, 1H ), 4.67 (dd, *J* = 7.3, 2.7 Hz, 1H), 7.10-7.16 (m, 2H), 7.22-7.32 (m, 2H), 8.40-8.46 (m, 1H). ¹³C NMR (126 MHz, CDCl₃) δ 14.2, 31.0, 32.2, 32.4, 37.5, 50.0, 50.1, 60.9, 61.2, 115.2, 115.3, 115.4, 124.3, 129.1, 129.2, 129.8, 130.0, 159.8, 161.7, 167.9, 168.0, 171.4.

### Compound (2R)-6: (2R)-2-(3-(dimethylamino)-2,5-dioxopyrrolidin-1-yl)-N-(2-fluorobenzyl)propanamidesuccinate

White solid: mp 119.6-120.4°C; C₃₆H₄₆F₂N₆O₁₀ (760.79), monoisotope mass: 321.15. UPLC (purity: > 99.9%): t_{R} = 2.93 min, (M + H)⁺ 322.2. ¹H NMR (500 MHz, CDCl₃) δ 1.45-1.51 (m, 3H), 2.34-2.48 (m, 2H), 2.52-2.71 (m, 2H), 2.95 (s, 6H), 3.05-3.31 (m, 3H), 4.33-4.45 (m, 2H), 4.81 (dd, *J* = 11.0, 7.3 Hz, 1H), 6.82-7.12 (m, 2H), 7.18-7.35 (m, 1H), 7.43-7.59 (m, 1H), 8.08 (br. s, 1H). ¹³C NMR (126 MHz, CDCl₃) δ 14.2, 31.0, 32.2, 32.4, 32.6, 37.5, 50.0, 50.1, 60.9, 61.2, 115.2, 115.3, 115.4, 124.3, 129.1, 129.2, 129.8, 130.0, 159.8, 161.7, 167.9, 168.0, 171.4 , 176.5, 177.4.

### Compound (2S)-6: (2S)-2-(3-(dimethylamino)-2,5-dioxopyrrolidin-1-yl)-N-(2-fluorobenzyl)propanamide succinate

White solid: mp 118.8-119.4°C; C₃₆H₄₆F₂N₆O₁₀ (760.79), monoisotope mass: 321.15. UPLC (purity: > 99.9%): t_{R} = 2.92 min, (M + H)⁺ 322.2. ¹H NMR (500 MHz, CDCl₃) δ 1.44-1.50 (m, 3H), 2.32-2.45 (m, 2H), 2.51-2.74 (m, 2H), 2.97 (s, 6H), 3.02-3.35 (m, 3H), 4.31-4.42 (m, 2H), 4.81 (dd, *J* = 11.0, 7.3 Hz, 1H), 6.85-7.36 (m, 3H), 7.41-7.59 (m, 1H), 8.09 (br. s, 1H). ¹³C NMR (126 MHz, CDCl₃) δ 14.1, 31.0, 32.1, 32.4, 32.6, 37.5, 50.0, 50.1, 60.8, 61.2, 115.2, 115.3, 115.4, 124.3, 129.1, 129.2, 129.7, 130.0, 159.8, 161.7, 167.9, 168.0, 171.4, 176.5, 177.4.

### Compound (2R,S)-6: (2R,S)-2-(3-(dimethylamino)-2,5-dioxopyrrolidin-1-yl)-N-(2-fluorobenzyl)propanamidesuccinate

White solid: mp 106.1-106.7°C; C₃₆H₄₆F₂N₆O₁₀ (760.79), monoisotope mass: 321.15. UPLC (purity: > 99.9%): t_{R} = 2.93 min, (M + H)⁺ 322.2. ¹H NMR (500 MHz, CDCl₃) δ 1.44-1.50 (m, 3H), 2.32-2.49 (m, 2H), 2.52-2.73 (m, 2H), 2.96 (s, 6H), 3.02-3.34 (m, 3H), 4.31-4.41 (m, 2H), 4.82 (dd, *J* = 11.0, 7.3 Hz, 1H), 6.85-7.37 (m, 3H), 7.41-7.58 (m, 1H), 8.07 (br. s, 1H). ¹³C NMR (126 MHz, CDCl₃) δ 14.1, 31.0, 32.1, 32.4, 32.6, 37.5, 50.0, 50.1, 60.8, 61.2, 115.2, 115.3, 115.4, 124.3, 129.1, 129.2, 129.7, 130.0, 159.8, 161.7, 167.9, 168.0, 171.4, 176.5, 177.4.

### Compound (2R)-6: (2R)-2-(3-(dimethylamino)-2,5-dioxopyrrolidin-1-yl)-N-(2-fluorobenzyl)propanamide fumarate

White solid: mp 118.3-119.1°C; C₃₆H₄₄F₂N₆O₁₀ (758.78), Monoisotopic mass: 321.15. UPLC (purity: > 99.9%): t_{R} = 2.91 min, (M + H)⁺ 322.2. ¹H NMR (500 MHz, CDCl₃) δ 1.42-1.49 (m, 3H), 2.97 (s, 6H), 3.08-3.31 (m, 2H), 3.42-3.58 (m, 2H), 4.57-4.62 (m, 2H), 4.78 (dd, *J* = 14.5, 7.5 Hz, 1H), 6.97 (t, *J* = 9, 5 Hz, 1H), 7.02-7.35 (m, 3H), 7.44-7.59 (m, 1H), 7.95 (br. s, 1H). ¹³C NMR (126 MHz, CDCl₃) δ 14.1, 14.2, 31.0, 32.2, 32.4, 37.5, 50.0, 50.1, 60.9, 61.2, 115.2, 115.3, 115.4, 124.3, 125.2, 129.0, 129.1, 129.2, 129.8, 130.0, 134.2, 138.9, 159.7 , 161.7, 167.9, 168.0, 170.5, 171.3.

### Compound (2S)-6: (2S)-2-(3-(dimethylamino)-2,5-dioxopyrrolidin-1-yl)-N-(2-fluorobenzyl)propanamide fumarate

White solid: mp 118.1-118.8°C; C₃₆H₄₄F₂N₆O₁₀ (758.78), monoisotopic mass: 321.15. UPLC (purity: > 99.9%): t_{R} = 2.92 min, (M + H)⁺ 322.2. ¹H NMR (500 MHz, CDCl₃) δ 1.41-1.47 (m, 3H), 2.96 (s, 6H), 3.07-3.29 (m, 2H), 3.41-3.53 (m, 2H), 4.51-4.64 (m, 2H), 4.77 (dd, *J* = 14.5, 7.5 Hz, 1H), 6.91-6.98 (m, 1H), 7.05-7.39 (m, 3H), 7.46-7.63 (m, 1H), 7.99 (br. s, 1H). ¹³C NMR (126 MHz, CDCl₃) δ 14.1, 14.2, 31.1, 32.2, 32.4, 37.5, 50.0, 50.1, 60.9, 61.2, 115.2, 115.3, 115.4, 124.2, 125.2, 129.0, 129.1, 129.2, 129.8, 130.0, 134.2, 138.9, 159.7, 161.7, 167.9, 168.0, 170.5, 171.3.

### Compound (2R,S)-6: (2R,S)-2-(3-(dimethylamino)-2,5-dioxopyrrolidin-1-yl)-N-(2-fluorobenzyl)propanamide fumarate

White solid: mp 104.7-105.4°C; C₃₆H₄₄F₂N₆O₁₀ (758.78), monoisotopic mass: 321.15. UPLC (purity: > 99.9%): t_{R} = 2.90 min, (M + H)⁺ 322.2. ¹H NMR (500 MHz, CDCl₃) δ 1.43-1.49 (m, 3H), 2.98 (s, 6H), 3.05-3.27 (m, 2H), 3.47-3.59 (m, 2H), 4.52-4.68 (m, 2H), 4.78 (dd, *J* = 14.5, 7.5 Hz, 1H), 6.91-6.97 (m, 1H), 6.99-7.33 (m, 3H), 7.41-7.58 (m, 1H), 8.02 (br. s, 1H). ¹³C NMR (126 MHz, CDCl₃) δ 14.1, 14.2, 31.1, 32.2, 32.4, 37.5, 50.0, 50.1, 60.9, 61.3, 115.2, 115.3, 115.5, 124.2, 125.1, 129.0, 129.1, 129.2, 129.8, 130.0, 134.2, 138.7, 159.7, 161.7, 167.9, 168.0, 170.5, 171.3.

### Example 2. Biological activity of the compounds according to the invention

### Anticonvulsant activity studies

The studies were carried out on male white Swiss mice (CD-1) weighing 18-26 g. All procedures were performed in accordance with the applicable Polish and international guidelines for the ethics of animal research, after obtaining of the appropriate institutional consent. Substances **(2*R,S*)-3, (2*R*)-3, (2*S*)-3, (2*R,S*)-4, (2*R*)-4, (2*S*)-4** and monohydrochlorides **(2*R,S*)-5, (2*R*)-5, (2*S*)-5, (2*R,S*)-6, (2*R*)-6, (2*S*)-6** were administered intraperitoneally (*ip*), after dissolution in a mixture of DMSO, PEG-400, water for injection (10/40/50, *v*/*v*/*v*), as a single injections of 10 mL/kg volume, 30 minutes prior to the test. Screening tests were performed in groups consisted of 4 mice. All tests were performed according to the procedures described in the specialist literature: maximal electroshock test (MES) (Kamiński et al. Bioorg. Med. Chem. 2015, 23, 2548-2561; Castel-Branco et al. Pharmacol. 2009, 31, 101-106, Riban et al. Neurosci. 2002, 112, 101-111); psychomotor seizure test (6 Hz, 32 mA) (Barton et al. Epilepsy Res. 2001, 47, 217-227; Wojda et al. Epilepsy Res. 2009, 86, 163-174); subcutaneous pentylenetetrazole test (scPTZ) (Ferreri et al. Pharmacol. Biochem. Behav. 2004, 77, 85-94). Results of the performed tests are presented in **Table 3.**

**Table 3. Results of the screening tests at dose of 100 mg/kg, for compounds covered by formula (I), and their chemical insoluble in water prototypes (2R,S)-1, (2R)-1, (2S)-1, (2R,S)-2, (2R)-2, (2S)-2, which were disclosed in the Polish patent application P.429656.**

| Compound | Test* | | |
|---|---|---|---|
| | MES | 6 Hz (32 mA) | *sc*PTZ |
| **(2*R,S*)-3** | 3/4 | 4/4 | 2/4 |
| **(2*R*)-3** | 4/4 | 4/4 | 3/4 |
| **(2*S*)-3** | 2/4 | 2/4 | 2/4 |
| **(2*R,S*)-4** | 3/4 | 3/4 | 2/4 |
| **(2*R*)-4** | 4/4 | 4/4 | 3/4 |
| **(2*S*)-4** | 2/4 | 2/4 | 2/4 |
| **(2*R*,*S*)-5 × HCl** | 3/4 | 4/4 | 2/4 |
| **(2*R*)-5 × HCl** | 3/4 | 4/4 | 3/4 |
| **(2*S*)-5 × HCl** | 2/4 | 3/4 | 2/4 |
| **(2*R*,*S*)-6 × HCl** | 3/4 | 4/4 | 2/4 |
| **(2*R*)-6 × HCl** | 3/4 | 4/4 | 3/4 |
| **(2*S*)-6 × HCl** | 2/4 | 3/4 | 2/4 |
| **(2*R*,*S*)-1^{#}** | 3/4 | 3/4 | 2/4 |
| **(2*R*)-1^{#}** | 3/4 | 4/4 | 3/4 |
| **(2*S*)-1^{#}** | 1/4 | 4/4 | 2/4 |
| **(2*R*,*S*)-2^{#}** | 3/4 | 3/4 | 3/4 |
| **(2*R*)-2^{#}** | 4/4 | 4/4 | 3/4 |
| **(2*S*)-2^{#}** | 2/4 | 2/4 | 2/4 |

| | | | |
|---|---|---|---|
| *Tests carried out at the time point of 0.5 h in mice after intraperitoneal administration. The data indicate the number of mice protected in a given seizure model/number of mice tested; MES - maximal electroshock test; 6 Hz (32 mA) - psychomotor seizure test of convulsions induced by a low frequency current (6 Hz) and intensity of 32 mA; scPTZ test - convulsions induced by subcutaneous administration of pentetrazole. ^{#}Substances disclosed in the Polish patent application P.429656. | | | |

The obtained *in vivo* results in mice at a screening dose of 100 mg/kg (intraperitoneal administration) showed that compounds **(2*R,S*)-3, (2R)-3, (2*S*)-3, (2*R,S*)-4, (2*R*)-4, (2*S*)-4** and monohydrochlorides **(2R,5)-5, (2R)-5, (2*S*)-5, (2R,5)-6, (2R)-6, (2*S*)-6,** described by the general formula (**I**), have broad anticonvulsant activity in various animal models of epileptic seizures, i.e. in the maximal electroshock test (MES), the pentetrazole subcutaneous seizure test (scPTZ), and the 6 Hz (32 mA) model, protecting 25-100% of the animals tested (**Table 3**). These models are recognized as the most important and widely used pharmacological tests allowing the identification of candidates for new antiepileptic drugs. Substances exhibiting protective activity in the aforementioned seizure models, can therefore be potentially effective in various types of human epilepsy; including tonic-clonic seizures with or without secondary generalization, generalized absence seizures, myoclonic seizures, partial seizures, and drug-resistant epilepsy. An advantageous feature of the compounds of formula (**I**) is that they are effective in all of the aforementioned seizure tests, i.e. MES, scPTZ and 6 Hz (32 mA). Advantageously, the disclosed broad spectrum of protective activity in the *in vivo* studies, confirms the possibility of using the compounds of the invention in monotherapy of various forms of epilepsy in humans, instead of polytherapy, which currently is the primary pharmacotherapeutic method used in epilepsy. The screening data shown in **Table 3** indicates potent anticonvulsant activity of the *R*-enantiomers, i.e. compounds **(2*R*)-3, (2*R*)-4, (2*R*)-5** i **(2*R*)-6.** In majority of cases, these compounds provided 100% protection in all models of epileptic seizures used. Therefore, the*R*- absolute configuration of the chiral carbon atom in the propanamide moiety seems to be preferential for anticonvulsant activity of compounds tested herein. Another advantage of the compounds of formula (**I**), especially substances obtained in the form of salts (e.g. monohydrochlorides), i.e. **(2*R,S*)-5, (2*R*), (2*S*)-5, (2*R,S*)-6, (2*R*)-6, (2*S*)-6,** results from their very good solubility in water. Due to the solubility, the preparations could be administered intravenously, in order to obtain a rapid effect of the treatment. This is crucial in situations requiring immediate intervention to stop seizures, including, e.g status epilpepticus, which is considered as a life-threatening situation. In addition to the improved solubility and the resulting advantages in medical applications, the compounds constituting the subject of this application, and the general structure of which is represented by formula (**I**), unexpectedly, despite significant chemical modifications relying on the removal of one of the carbonyl groups in the pyrrolidine-2,5-dione ring, or introducing the dimethylamine fragment into the aforementioned core structure, what lead to significant changes in geometry and polarity of the molecules, have almost identical or potent anticonvulsant activity as their chemical prototypes **(2*R*,*S*)-1, (2*R*)-1, (2*S*)-1, (2*R,S*)-2, (2*R*)-2, (2*S*)-2,** which were disclosed in the application Polish patent P.429656 (**Table 3**). Additionally, unexpectedly, new pyrrolidin-2-one derivatives, i.e. **(2R,*S*)-3, (2*R*)-3, (2*S*)-3, (2*R,S*)-4** , **(2*R*)-4,** and **(2*S*)-4** exhibit protective activity in the MES and scPTZ tests unlike to model AED - levetiracetam with structure based on the same heterocyclic ring, but which remains inactive in the aforementioned models of epileptic seizures. Moreover, the introduction of a sterically big dimethylamine fragment at the 3-position of the pyrrolidine-2,5-dione ring, unexpectedly does not lead to a significant decrease in biological activity, at least in the case of derivatives **(2*R,S*)-5, (2*R*)-5, (2*S*)-5, (2*R*,*S*)-6, (2*R*)-6** and **(2*S*)-6.** It should be emphasized that the presence of one or two methyl groups at the 3-position of close analogs of the present compounds, resulted in decrease in anticonvulsant effect, as it was proved in our earlier research (Kaminski, et al. Bioorg. Med. Chem. 2015, 23, 2548-2561).

### Embodiments of the invention

**1.** Compound of formula **(I):** 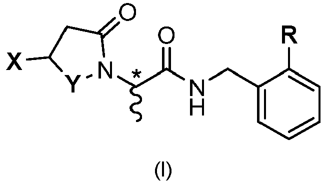 wherein:
   **X** represents hydrogen or N(CH₃)₂,
   **Y** represents CH₂ or C=O,
   **R** represents hydrogen or a halogen atom, preferably F,
   wherein, when **Y** is C=O, then **X** represents N(CH₃)₂,
   or its optical isomers, mixtures thereof, and pharmaceutically acceptable salts.
**2.** Compound according to embodiment 1, wherein it is selected from a group comprising:
   *N*-benzyl-2-(2-oxopyrrolidin-1-yl)propanamide,
   *N*-(2-fluorobenzyl)-2-(2-oxopyrrolidin-1-yl)propanamide,
   *N*-benzyl-2-(3-(dimethylamino)-2,5-dioxopyrrolidin-1-yl)propanamide,
   and 2-(3-(dimethylamino)-2,5- dioxopyrrolidin-1-yl)-*N*-(2-fluorobenzyl)propanamide.
**3.** Compound according to embodiment 1 or 2, wherein, it is selected from the group comprising: N-benzyl-2-(3-(dimethylamino)2,5-dioxopyrrolidin-1-yl)propanamide, and 2-(3-(dimethylamino)-2,5-dioxopyrrolidin-1-yl)-*N*-(2-fluorobenzyl)propanamide.
**4.** Compound according to any of the previous embodiments, wherein it is in a form of pharmaceutically acceptable salt, selected from: hydrochloride, sulfate, methanesulfonate, toluenesulfonate, succinate, fumarate, or lactate.
**5.** Compound according to any of the previous embodiments, wherein it is selected from a group comprising:
   (2*R*)-*N*-benzyl-2-(2-oxopyrrolidin-1-yl)propanamide,
   (2*S*)-*N*-benzyl-2-(2-oxopyrrolidin-1-yl)propanamide,
   (2*R*,*S*)-N-benzyl-2-(2-oxopyrrolidin-1-yl)propanamide,
   (2*R*)-*N*-(2-fluorobenzyl)-2-(2-oxopyrrolidin-1-yl)propanamide,
   (2*S*)-*N*-(2-fluorobenzyl)-2-(2-oxopyrrolidin-1-yl)propanamide,
   (2*R*,*S*)-N-(2-fluorobenzyl)-2-(2-oxopyrrolidin-1-yl)propanamide,
   (2R)-*N*-benzyl-2-(3-(dimethylamino)-2,5-dioxopyrrolidin-1-yl)propanamide hydrochloride,
   (*2S*)*-N*-benzyl-2-(3-(dimethylamino)-2,5-dioxopyrrolidin-1-yl)propanamide hydrochloride,
   (*2R,S*)*-N*-benzyl-2-(3-(dimethylamino)-2,5-dioxopyrrolidin-1-yl)propanamide hydrochloride,
   (*2R*)-2-(3-(dimethylamino)-2,5-dioxopyrrolidin-1-yl)-*N*-(2-fluorobenzyl)propanamide hydrochloride,
   (*2S*)-2-(3-(dimethylamino)-2,5-dioxopyrrolidin-1-yl)-*N*-(2-fluorobenzyl)propanamide hydrochloride,
   (*2R,S*)-2-(3-(dimethylamino)-2,5-dioxopyrrolidin-1-yl)-*N*-(2-fluorobenzyl)propanamide hydrochloride,
   (*2R*)-*N*-benzyl-2-(3-(dimethylamino)-2,5-dioxopyrrolidin-1-yl)propanamide metanesulfonate,
   (*2S*)-*N*-benzyl-2-(3-(dimethylamino)-2,5-dioxopyrrolidin-1-yl)propanamide metanesulfonate,
   (2*R,S*)*-N-*benzyl-2-(3-(dimethylamino)-2,5-dioxopyrrolidin-1-yl)propanamide metanesulfonate,
   (*2R*)-*N*-benzyl-2-(3-(dimethylamino)-2,5-dioxopyrrolidin-1-yl)propanamide toluenesulfonate,
   (*2S*)-*N*-benzyl-2-(3-(dimethylamino)-2,5-dioxopyrrolidin-1-yl)propanamide toluenesulfonate,
   (*2R,S*)*-N-*benzyl-2-(3-(dimethylamino)-2,5-dioxopyrrolidin-1-yl)propanamide toluenesulfonate,
   (2*R*)-*N*-benzyl-2-(3-(dimethylamino)-2,5-dioxopyrrolidin-1-yl)propanamide lactate,
   (*2S*)*-N-*benzyl-2-(3-(dimethylamino)-2,5-dioxopyrrolidin-1-yl)propanamide lactate,
   (*2R,S*)*-N*-benzyl-2-(3-(dimethylamino)-2,5-dioxopyrrolidin-1-yl)propanamide lactate,
   (*2R*)-*N*-benzyl-2-(3-(dimethylamino)-2,5-dioxopyrrolidin-1-yl)propanamide sulfate,
   (*2S*)-*N*-benzyl-2-(3-(dimethylamino)-2,5-dioxopyrrolidin-1-yl)propanamide sulfate,
   (*2R,S)-N*-benzyl-2-(3-(dimethylamino)-2,5-dioxopyrrolidin-1-yl)propanamide sulfate,
   (*2R*)-N-benzyl-2-(3-(dimethylamino)-2,5-dioxopyrrolidin-1-yl)propanamide succinate,
   (*2S*)-N-benzyl-2-(3-(dimethylamino)-2,5-dioxopyrrolidin-1-yl)propanamide succinate,
   (*2R,S*)*-N-*benzyl-2-(3-(dimethylamino)**-**2,5-dioxopyrrolidin-1-yl)propanamide succinate,
   (*2R*)-*N*-benzyl-2-(3-(dimethylamino)-2,5-dioxopyrrolidin-1-yl)propanamide fumarate,
   (*2S*)-*N*-benzyl-2-(3-(dimethylamino)-2,5-dioxopyrrolidin-1-yl)propanamide fumarate,
   (*2R,S*)*-N*-benzyl-2-(3-(dimethylamino)-2,5-dioxopyrrolidin-1-yl)propanamide fumarate,
   (*2R*)-2-(3-(dimethylamino)-2,5-dioxopyrrolidin-1-yl)-*N*-(2-fluorobenzyl)propanamide methanesulfonate,
   (*2S*)-2-(3-(dimethylamino)-2,5-dioxopyrrolidin-1-yl)-*N*-(2-fluorobenzyl)propanamide methanesulfonate,
   (*2R,S*)-2-(3-(dimethylamino)-2,5-dioxopyrrolidin-1-yl)-*N*-(2-fluorobenzyl)propanamide methanesulfonate,
   (*2R*)-2-(3-(dimethylamino)-2,5-dioxopyrrolidin-1-yl)-*N*-(2-fluorobenzyl)propanamide toluenesulfonate,
   (*2S*)-2-(3-(dimethylamino)-2,5-dioxopyrrolidin-1-yl)-*N*-(2-fluorobenzyl)propanamide toluenesulfonate,
   (*2R,S*)-2-(3-(dimethylamino)-2,5-dioxopyrrolidin-1-yl)-*N*-(2-fluorobenzyl)propanamide toluenesulfonate,
   (*2R*)-2-(3-(dimethylamino)-2,5-dioxopyrrolidin-1-yl)-*N*-(2-fluorobenzyl)propanamide lactate,
   (*2S*)-2-(3-(dimethylamino)-2,5-dioxopyrrolidin-1-yl)-*N*-(2-fluorobenzyl)propanamide lactate,
   (*2R,S*)-2-(3-(dimethylamino)-2,5-dioxopyrrolidin-1-yl)-*N*-(2-fluorobenzyl)propanamide lactate,
   (*2R*)-2-(3-(dimethylamino)-2,5-dioxopyrrolidin-1-yl)-*N*-(2-fluorobenzyl)propanamide sulfate,
   (*2S*)-2-(3-(dimethylamino)-2,5-dioxopyrrolidin-1-yl)-*N*-(2-fluorobenzyl)propanamide sulfate,
   (*2R,S*)-2-(3-(dimethylamino)-2,5-dioxopyrrolidin-1-yl)-*N*-(2-fluorobenzyl)propanamide sulfate,
   (*2R*)-2-(3-(dimethylamino)-2,5-dioxopyrrolidin-1-yl)-*N*-(2-fluorobenzyl)propanamide succinate,
   (*2S*)-2-(3-(dimethylamino)-2,5-dioxopyrrolidin-1-yl)-*N*-(2-fluorobenzyl)propanamide succinate,
   (*2R,S*)-2-(3-(dimethylamino)-2,5-dioxopyrrolidin-1-yl)-*N*-(2-fluorobenzyl)propanamide succinate,
   (*2R*)-2-(3-(dimethylamino)-2,5-dioxopyrrolidin-1-yl)-*N*-(2-fluorobenzyl)propanamide fumarate,
   (*2S*)-2-(3-(dimethylamino)-2,5-dioxopyrrolidin-1-yl)-*N*-(2-fluorobenzyl)propanamide fumarate, and
   (*2R,S*)-2-(3-(dimethylamino)-2,5-dioxopyrrolidin-1-yl)-*N*-(2-fluorobenzyl)propanamide fumarate.

## Claims

1. A compound of formula (I): 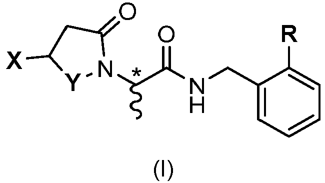 wherein:
**X** represents N(CH₃)₂,
**Y** represents C=O,
**R** represents hydrogen or a halogen atom,
or its optical isomers, mixtures thereof, and pharmaceutically acceptable salts.

2. A compound according to claim 1, wherein **R** is a halogen, and optionally **R** is F.

3. A compound according to claim 1 or 2, wherein the compound is N-benzyl-2-(3-(dimethylamino)2,5-dioxopyrrolidin-1-yl)propanamide or 2-(3-(dimethylamino)-2,5-dioxopyrrolidin-1-yl)-*N*-(2-fluorobenzyl)propanamide.

4. A compound according to any of the preceding claims, wherein the compound is the R-enantiomer.

5. A compound according to claim 4, wherein the compound is (2R)-N-benzyl-2-(3-(dimethylamino)2,5-dioxopyrrolidin-1-yl)propanamide or (2R)-2-(3-(dimethylamino)-2,5-dioxopyrrolidin-1-yl)-*N*-(2-fluorobenzyl)propanamide.

6. A compound according to any of the preceding claims wherein the compound is in a form of pharmaceutically acceptable salt, selected from: hydrochloride, sulfate, methanesulfonate, toluenesulfonate, succinate, fumarate, or lactate.

7. A compound according to claim 6, wherein the compound is selected from a group comprising:
(2*R*)-*N*-benzyl-2-(3-(dimethylamino)-2,5-dioxopyrrolidin-1-yl)propanamide hydrochloride,
(2*R*)-*N*-benzyl-2-(3-(dimethylamino)-2,5-dioxopyrrolidin-1-yl)propanamide metanesulfonate,
(2*R*)-*N*-benzyl-2-(3-(dimethylamino)-2,5-dioxopyrrolidin-1-yl)propanamide toluenesulfonate,
(2*R*)-*N*-benzyl-2-(3-(dimethylamino)-2,5-dioxopyrrolidin-1-yl)propanamide lactate,
(2*R*)-*N*-benzyl-2-(3-(dimethylamino)-2,5-dioxopyrrolidin-1-yl)propanamide sulfate,
(2*R*)-*N*-benzyl-2-(3-(dimethylamino)-2,5-dioxopyrrolidin-1-yl)propanamide succinate, and
(2*R*)-*N*-benzyl-2-(3-(dimethylamino)-2,5-dioxopyrrolidin-1-yl)propanamide fumarate.

8. A compound according to claim 6, wherein the compound is selected from a group comprising:
(2*R*)-2-(3-(dimethylamino)-2,5-dioxopyrrolidin-1-yl)-*N*-(2-fluorobenzyl)propanamide hydrochloride,
(2*R*)-2-(3-(dimethylamino)-2,5-dioxopyrrolidin-1-yl)-*N*-(2-fluorobenzyl)propanamide methanesulfonate,
(2*R*)-2-(3-(dimethylamino)-2,5-dioxopyrrolidin-1-yl)-*N*-(2-fluorobenzyl)propanamide toluenesulfonate,
(2*R*)-2-(3-(dimethylamino)-2,5-dioxopyrrolidin-1-yl)-*N*-(2-fluorobenzyl)propanamide lactate,
(2*R*)-2-(3-(dimethylamino)-2,5-dioxopyrrolidin-1-yl)-*N*-(2-fluorobenzyl)propanamide sulfate,
(2*R*)-2-(3-(dimethylamino)-2,5-dioxopyrrolidin-1-yl)-*N*-(2-fluorobenzyl)propanamide succinate, and
(2*R*)-2-(3-(dimethylamino)-2,5-dioxopyrrolidin-1-yl)-*N*-(2-fluorobenzyl)propanamide fumarate.

9. A compound according to any of the preceding claims for use in therapy.

10. A compound according to any of claims 1 to 8 for use in the treatment of epilepsy.

11. The compound for the use according to claim 10 wherein the epilepsy is selected from tonic-clonic seizures with or without secondary generalization, generalized absence seizures, myoclonic seizures, partial seizures, and drug-resistant epilepsy.

12. the compound for the use according to claim 10 or 11, wherein the compound is used as a monotherapy in the treatment of epilepsy.
